(19) Europäisches Patentamt
European Patent Office
Office européen des brevets

(11) EP 1 256 582 A1

(12) EUROPEAN PATENT APPLICATION
published in accordance with Art. 158(3) EPC

(43) Date of publication:
**13.11.2002 Bulletin 2002/46**

(21) Application number: **01902774.7**

(22) Date of filing: **06.02.2001**

(51) Int Cl.[7]: **C07D 471/04**, A61K 31/437, A61P 3/10, A61P 11/06, A61P 17/00, A61P 25/00, A61P 29/00, A61P 31/04, A61P 37/06, A61P 37/08, A61P 43/00

(86) International application number:
**PCT/JP01/00816**

(87) International publication number:
**WO 01/058900 (16.08.2001 Gazette 2001/33)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **09.02.2000 JP 2000031270**
**13.09.2000 JP 2000277507**

(71) Applicant: **HOKURIKU SEIYAKU CO., LTD.**
**Katsuyama-shi, Fukui 911-0813 (JP)**

(72) Inventors:
- **KATO, Hideo, HOKURIKU SEIYAKU CO., LTD.**
  **Katsuyama-shi, Fukui 911-0813 (JP)**
- **SAKAGUCHI, Jun,**
  **HOKURIKU SEIYAKU CO., LTD.**
  **Katsuyama-shi, Fukui 911-0813 (JP)**
- **IZUMI, Tomoyuki,**
  **HOKURIKU SEIYAKU CO., LTD.**
  **Katsuyama-shi, Fukui 911-0813 (JP)**
- **KATO, Ken-ichi, HOKURIKU SEIYAKU CO., LTD.**
  **Katsuyama-shi, Fukui 911-0813 (JP)**

(74) Representative:
**Sternagel, Fleischer, Godemeyer & Partner**
**Patentanwälte**
**An den Gärten 7**
**51491 Overath (DE)**

(54) **1H-IMIDAZOPYRIDINE DERIVATIVES**

(57) A 1H-imidazopyridine derivative represented by the following general formula (I) or a salt thereof having an inhibitory action against production of a cytokine:

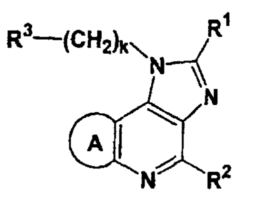

(I)

wherein $R^1$ represents hydrogen atom, an alkyl group, a cycloalkyl group, or an aryl group; $R^2$ represents a cycloalkyl group, an alkyl group, an aryl group, cyano group, mercapto group, carboxyl group, or carbamoyl group; ring A represents a homocyclic or heterocyclic ring; $R^3$ represents an amino group or a saturated nitrogen-containing heterocyclic group; and k represents an integer of from 0 to 3; provided that the compound wherein $R^3$ represents a saturated nitrogen-containing heterocyclic group and $R^2$ is a non-substituted alkyl group is excluded.

EP 1 256 582 A1

## Description

### Technical Field

The present invention relates to novel 1H-imidazopyridine derivatives or salts thereof which have a potent inhibitory action against production of tumor necrotizing factor (TNF) or interleukin-1 (IL-1) and are useful as active ingredients of medicaments.

### Background Art

Some compounds having 1H-imidazoquinoline structure are known which are analogous to the compounds of the present invention. For example, Journal of Medicinal Chemistry, Vol. 11, p. 87 (1968) discloses 1-(2-piperidinoethyl)-1H-imidazo-[4,5-c]quinoline, Japanese Patent Unexamined Publication (KOKAI) No. Sho 60-123488/1985 discloses 1-isobutyl-1H-imidazo[4,5-c]quinoline-4-amine (general name: imiquimod) as a compound having an antiviral action, and Hungarian Patent Publication No. 34479 (Patent No. 190109) discloses 1-(2-diethylamino-ethyl)-1H-imidazo[4,5-c]quinoline as a compound having analgesic and anticonvulsant actions. However, 1H-imidazopyridine derivatives as those according to the present invention have not been known so far.

The aforementioned imiquimod has been known to have an inducing action of a few kinds of cytokines such as interferon (IFN), TNF, IL-1 and the like, which is described in Journal of Interferon Research, Vol. 14, p. 81 (1994). In addition, as compounds having IFN-inducing activity, International Publication WO99/29693 discloses imidazonaphthylidine derivatives and Japanese Patent Unexamined Publication (KOKAI) No. Hei11-80156/1999 discloses imidazopyridine derivatives. However, 1H-imidazopyridine derivatives or 1H-imidazoquinoline derivatives having an inhibitory action against production of TNF or IL-1, which action is totally opposite to those taught by the aforementioned prior arts, have not been known so far.

### Disclosure of the Invention

An object of the present invention is to provide novel compounds which have excellent inhibitory actions against production of cytokines such as TNF, IL-1 and the like and are useful as medicaments.

The inventors of the present invention made intensive studies to achieve the object. As a result, they found novel 1H-imidazopyridine derivatives which have an excellent inhibitory action against production of TNF or IL-1 and achieved the present invention.

The present invention thus relates to novel 1H-imidazopyridine derivatives represented by the following general formula (I) or salts thereof:

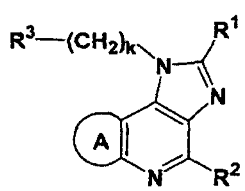

(I)

wherein $R^1$ represents hydrogen atom, an alkyl group which may be substituted, a cycloalkyl group which may be substituted, or an aryl group which may be substituted; $R^2$ represents a cycloalkyl group which may be substituted, an alkyl group which may be substituted, an aryl group which may be substituted, cyano group, mercapto group, carboxyl group, or carbamoyl group; ring A represents a homocyclic or heterocyclic ring which may be substituted; $R^3$ represents an amino group which may be substituted or a saturated nitrogen-containing heterocyclic group which may be substituted; and k represents an integer of from 0 to 3; provided that the compound wherein $R^3$ represents a saturated nitrogen-containing heterocyclic group which may be substituted and $R^2$ is a non-substituted alkyl group is excluded.

According to a preferred embodiment of the present invention, provided are novel 1H-imidazopyridine derivatives represented by the following general formula (II) or salts thereof:

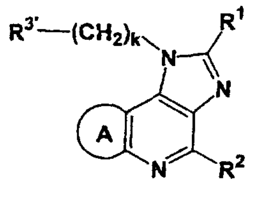

(II)

wherein $R^1$, $R^2$, ring A and k have the same meanings as those defined above; $R^{3'}$ represents a group represented by the following formula (III)

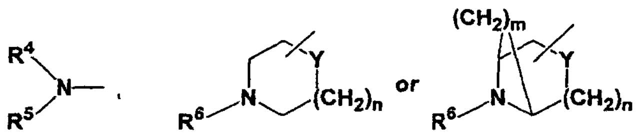

, or (III)

$R^4$, $R^5$, $R^6$ may be the same or different and represent hydrogen atom, an alkyl group which may be substituted, a benzyl group which may be substituted, triphenylmethyl group, an acyl group which may be substituted, an alkoxycarbonyl group which may be substituted, a benzyloxycarbonyl group which may be substituted, a thiocarbamoyl group which may be substituted, an alkanesulfonyl group which may be substituted, a benzenesulfonyl group which may be substituted, or an amidino group which may be substituted; Y represents oxygen atom, sulfur atom, or nitrogen atom, a group represented by $CH_2$, CH, or NH, or a single bond; and m and n may be the same or different and represent an integer of from 0 to 2, provided that when $R^{3'}$ represents a group represented by the following formula (IV):

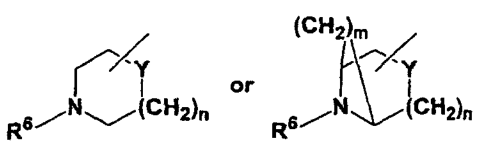

or (IV)

$R^2$ does not represent a non-substituted alkyl group. The compound represented by the aforementioned general formula (II) fall within the scope of the aforementioned general formula (I), i.e., they are characterized to have, as $R^3$ of the aforementioned general formula (I), the amino group which may have a specific substituent or the saturated nitrogen-containing heterocyclic group which may have a specific substituent represented by $R^{3'}$.

According to another preferred embodiment of the present invention, provided are the compounds or salts thereof represented by the aforementioned general formula (I) and formula (II) wherein the ring A is a benzene ring which may be substituted or a thiophene ring which may be substituted.

According to still another preferred embodiment, provided are the compounds or salts thereof represented by the aforementioned general formula (I) and formula (II) wherein $R^2$ is trifluoromethyl group.

From another aspect, there is provided a medicament which comprises the compound represented by the aforementioned general formula (I) or (II), or a pharmacologically acceptable salt thereof as an active ingredient. The medicament is useful for preventive and/or therapeutic treatment of diseases of humans and animals, in which a cytokine such as TNF or IL-1 is involved, which include chronic inflammatory diseases (e.g., rheumatic arthritis, osteoarthritis and the like), allergic rhinitis, atopic dermatitis, contact dermatitis, urticaria, eczema, pruritus cutaneus, prurigo, asthma, sepsis, septic shock, various autoimmune diseases [autoimmune hemic diseases (e.g., hemolytic anemia, anaplastic anemia, idiopathic thrombocythemia and the like), autoimmune intestinal diseases (e.g., ulcerative colitis, Crohn's disease and the like), autoimmune corneitis (e.g., keratoconjunctivitis sicca, spring catarrh and the like), endocrine ophthalmopathy, Graves disease, sarcoid granuloma, multiple sclerosis, systemic erythematodes, multiple chondritis, pachydermia, active chronic hepatitis, myasthenia gravis, psoriasis, interstitial pulmonary fibrosis and the like], diabetes, cancerous cachexia, HIV-infectious cachexia and the like.

According to a further aspect, there are provided an inhibitor against production of a cytokine which comprises

the compound represented by the aforementioned general formula (I) or (II), or a pharmacologically acceptable salt thereof as an active ingredient. As an preferred embodiment, provided is the inhibitor against production of tumor necrotizing factor (TNF) or interleukin-1 (IL-1).

Furthermore, according to the present invention, provided are a use of the compound represented by the aforementioned general formula (I) or (II), or a pharmacologically acceptable salt thereof for the manufacture of the aforementioned medicament; and a method for the preventive and/or therapeutic treatment of diseases in which a cytokine is involved, which comprises the step of administering a preventively and/or therapeutically effective amount of the compound represented by the aforementioned general formula (I) or (II) or a pharmacologically acceptable salt thereof to a mammal including a human.

Best Mode for Carrying Out the Invention

Specific explanations of the compounds of the aforementioned general formulas (I) and (II) of the present invention will be given below. In the aforementioned general formulas (I) and (II), examples of the alkyl group represented by $R^1$, $R^2$, $R^4$, $R^5$, and $R^6$ defined as an alkyl group which may be substituted, include, for example, methyl group, ethyl group, n-propyl group, isopropyl group, n-butyl group, isobutyl group, sec-butyl group, tert-butyl group, n-pentyl group, isopentyl group, neopentyl group, n-hexyl group and the like.

Examples of the cycloalkyl group represented by $R^1$ and $R^2$, defined as a cycloalkyl group which may be substituted, include, for example, cyclopropyl group, cyclobutyl group, cyclopentyl group, cyclohexyl group, cycloheptyl group and the like. Examples of the aryl group represented by $R^1$ and $R^2$, defined as an aryl group which may be substituted, include, for example, phenyl group, 2-pyridyl group, 3-pyridyl group, 4-pyridyl group, 3-pyridazinyl group, 4-pyridazinyl group, 2-pyrimidinyl group, 4-pyrimidinyl group, 5-pyrimidinyl group, pyrazinyl group, 2-furyl group, 3-furyl group, 2-thienyl group, 3-thienyl group, 1-pyrrolyl group, 2-pyrrolyl group, 3-pyrrolyl group, 1-imidazolyl group, 2-imidazolyl group, 4-imidazolyl group, 1-pyrazolyl group, 3-pyrazolyl group, 4-pyrazolyl group, 2-oxazolyl group, 4-oxazolyl group, 3-isoxazolyl group, 4-isoxazolyl group, 5-isoxazolyl group, 2-thiazolyl group, 4-thiazolyl group, 5-thiazolyl group, 3-isothiazolyl group, 4-isothiazolyl group, 5-isothiazolyl group, 1,2,3-triazol-1-yl group, 1,2,3-triazol-4-yl group, 1,2,3-triazol-5-yl group, 1,2,4-triazol-1-yl group, 1,2,4-triazol-3-yl group, 1,2,4-triazol-5-yl group, 1-tetrazolyl group, 5-tetrazolyl group, 1,2,5-thiadiazol-3-yl group, 1-naphthyl group, 2-naphthyl group, 2-quinolyl group, 3-quinolyl group, 4-quinolyl group, 1-indolyl group, 2-indolyl group, 3-indolyl group and the like.

Examples of the homocyclic or heterocyclic ring represented by ring A in the aforementioned general formulas (I) and (II), defined as a homocyclic or heterocyclic ring which may be substituted, include, for example, benzene ring, cyclopentene ring, cyclohexene ring, cycloheptene ring, cyclooctene ring, cycloheptadiene ring, thiophene ring, furan ring, pyridine ring, pyrazine ring, pyrimidine ring, pyrrole ring, thiazole ring, oxazole ring, azepine ring, naphthalene ring, quinoline ring and the like. Preferred ring includes benzene ring, thiophene ring or the like.

The saturated nitrogen-containing heterocyclic group represented by $R^3$ in the aforementioned general formula (I), which may be substituted, includes where $R^{3'}$ in the aforementioned general formula (II) is the saturated nitrogen-containing heterocyclic group represented by the general formula (IV) which may be substituted. The saturated nitrogen-containing heterocyclic group includes those having one or more nitrogen atoms as ring-constituting atom(s), and further optionally having one or more oxygen atoms or sulfur atoms as ring-constituting atom(s). Examples include 1-aziridinyl group, 2-aziridinyl group, 1-azetidinyl group, 2-azetidinyl group, 3-azetidinyl group, 1-pyrrolidinyl group, 2-pyrrolidinyl group, 3-pyrrolidinyl group, pyrazolidinyl group, imidazolidinyl group, piperidino group, 2-piperidyl group, 3-piperidyl group, 4-piperidyl group, 1-piperazinyl group, 2-piperazinyl group, hexahydro-1,2-diazin-3-yl group, hexahydro-1,3-diazin-2-yl group, hexahydro-1H-azepin-1-yl group, hexahydro-1H-azepin-2-yl group, hexahydro-1H-azepin-3-yl group, hexahydro-1H-azepin-4-yl group, hexahydro-1H-1,4-diazepin-1-yl group, hexahydro-1H-1,4-diazepin-2-yl group, hexahydro-1H-1,4-diazepin-5-yl group, hexahydro-1H-1,4-diazepin-6-yl group, 2-morpholinyl group, 3-morpholinyl group, morpholino group, 2-thiomorpholinyl group, 3-thiomorpholinyl group, 4-thiomorpholinyl group, 3-oxazolidinyl group, 3-isoxazolidinyl group, 3-thiazolidinyl group, 3-isothiazolidinyl group, tetrahydro-1,2,4-oxadiazol-3-yl group, tetrahydro-1,2,5-oxadiazol-3-yl group, 1,2,3-triazolidin-4-yl group, 1,2,4-triazolidin-3-yl group, tetrahydro-1,2,4-thiadiazol-3-yl group, 1,2,5-thiadiazolin-3-yl group, decahydroquinolyl group, 8-azabicyclo[3.2.1]octan-3-yl group, 9-azabicyclo[3.3.1]nonan-3-yl group and the like, and preferred groups include, for example, 3-piperidyl group, 4-piperidyl group, 1-piperazinyl group, 2-piperazinyl group, 3-pyrrolidinyl group, 2-azetidinyl group, 3-azetidinyl group, 2-morpholinyl group, 2-thiomorpholinyl group and the like.

In the aforementioned formula (II), examples of the acyl group represented by $R^4$, $R^5$, and $R^6$, defined as an acyl group which may be substituted, include, for example, formyl group, acetyl group, propionyl group, n-butyryl group, isobutyryl group, valeryl group, isovaleryl group, pivaloyl group, benzoyl group, 2-pyridylcarbonyl group, nicotinoyl group, isonicotinoyl group, 3-pyridazinylcarbonyl group, 4-pyridazinylcarbonyl group, 2-pyrimidinylcarbonyl group, 4-pyrimidinylcarbonyl group, 5-pyrimidinylcarbonyl group, pyrazinylcarbonyl group, 2-furylcarbonyl group, furoyl group, thenoyl group, 3-thienylcarbonyl group, 1-pyrrolylcarbonyl group, 2-pyrrolylcarbonyl group, 3-pyrrolylcarbonyl group,

1-imidazolylcarbonyl group, 2-imidazolylcarbonyl group, 4-imidazolylcarbonyl group, 1-pyrazolylcarbonyl group, 3-pyrazolylcarbonyl group, 4-pyrazolylcarbonyl group, 2-oxazolylcarbonyl group, 4-oxazolylcarbonyl group, 3-isoxazolylcarbonyl group, 4-isoxazolylcarbonyl group, 5-isoxazolylcarbonyl group, 2-thiazolylcarbonyl group, 4-thiazolylcarbonyl group, 5-thiazolylcarbonyl group, 3-isothiazolylcarbonyl group, 4-isothiazolylcarbonyl group, 5-isothiazolylcarbonyl group, 1,2,3-triazol-1-ylcarbonyl group, 1,2,3-triazol-4-ylcarbonyl group, 1,2,3-triazol-5-ylcarbonyl group, 1,2,4-triazol-1-ylcarbonyl group, 1,2,4-triazol-3-ylcarbonyl group, 1,2,4-triazol-5-ylcarbonyl group, 1-tetrazolylcarbonyl group, 5-tetrazolylcarbonyl group, 1,2,5-thiadiazol-3-ylcarbonyl group, 1-naphthoyl group, 2-naphthoyl group, 2-quinolylcarbonyl group, 3-quinolylcarbonyl group, 4-quinolylcarbonyl group, 1-indolylcarbonyl group, 2-indolylcarbonyl group, 3-indolylcarbonyl group, cyclohexylacetyl group, acryloyl group, phenylacetyl group and the like. Examples of the alkoxycarbonyl group represented by $R^4$, $R^5$, and $R^6$, defined as an alkoxycarbonyl group which may be substituted, include, for example, methoxycarbonyl group, ethoxycarbonyl group, n-propoxycarbonyl group, isopropoxycarbonyl group, n-butoxycarbonyl group, isobutoxycarbonyl group, sec-butoxycarbonyl group, tert-butoxycarbonyl group, n-pentyloxycarbonyl group, n-hexyloxycarbonyl group and the like. Examples of the alkanesulfonyl group represented by $R^4$, $R^5$, and $R^6$, defined as an alkanesulfonyl group which may be substituted, include, for example, methanesulfonyl group, ethanesulfonyl group, n-propanesulfonyl group, n-butanesulfonyl group and the like.

In the compounds of aforementioned general formulas (I) and (II) of the present invention, when certain functional groups are referred to as "which may be substituted," the substituent may be any group so long as it can substitute on the functional groups. The number, kind, and substituting position of the substituent are not particularly limited, and when two or more substituents exist, they may be the same or different. Examples of substitutable functional groups include halogen atoms such as fluorine atom, chlorine atom, bromine atom, or iodine atom; hydroxyl group; alkyl groups such as methyl group, ethyl group, n-propyl group, isopropyl group, n-butyl group, isobutyl group, sec-butyl group, tert-butyl group, n-pentyl group, isopentyl group, neopentyl group, and n-hexyl group; trifluoromethyl group; aryl groups such as phenyl group, naphthyl group, and pyridyl group; alkoxyl groups such as methoxy group, ethoxy group, n-propoxy group, isopropoxy group, n-butoxy group, isobutoxy group, sec-butoxy group, and tert-butoxy group; aryloxy groups such as phenoxy group, pyridyloxy group, naphthyloxy group; amino groups which may be substituted such as amino group, methylamino group, ethylamino group, n-propylamino group, isopropylamino group, cyclopropylamino group, cyclobutylamino group, cyclopentylamino group, cyclohexylamino group, dimethylamino group, diethylamino group, anilino group, pyridylamino group, benzylamino group, dibenzylamino group, acetylamino group, trifluoroacetylamino group, tert-butoxycarbonylamino group, benzyloxycarboxylamino group, benzhydrylamino group, and triphenylmethylamino group; acyl groups which may be substituted such as formyl group, acetyl group, propionyl group, n-butyryl group, isobutyryl group, valeryl group, isovaleryl group, pivaloyl group, fluoroacetyl group, difluoroacetyl group, trifluoroacetyl group, chloroacetyl group, dichloroacetyl group, and trichloroacetyl group; alkoxycarbonyl groups such as methoxycarbonyl group, ethoxycarbonyl group, n-propoxycarbonyl group, isopropoxycarbonyl group, n-butoxycarbonyl group, isobutoxycarbonyl group, sec-butoxycarbonyl group, tert-butoxycarbonyl group, n-pentyloxycarbonyl group, and n-hexyloxycarbonyl group; benzyloxycarbonyl group; carbamoyl group; alkylcarbamoyl groups such as methylcarbamoyl group, ethylcarbamoyl group, n-propylcarbamoyl group, isopropylcarbamoyl group, n-butylcarbamoyl group, isobutylcarbamoyl group, sec-butylcarbamoyl group, and tert-butylcarbamoyl group; thiocarbamoyl group; alkylthiocarbamoyl groups such as methylthiocarbamoyl group, ethylthiocarbamoyl group, n-propylthiocarbamoyl group, isopropylthiocarbamoyl group, n-butylthiocarbamoyl group, isobutylthiocarbamoyl group, sec-butylthiocarbamoyl group, and tert-butylthiocarbamoyl group; amidino group; alkylthio groups such as methylthio group, ethylthio group, and n-propylthio group; alkanesulfinyl groups such as methanesulfinyl group, ethanesulfinyl group, and n-propanesulfinyl group; alkanesulfonyl groups such as methanesulfonyl group, ethanesulfonyl group, n-propanesulfonyl group, and n-butanesulfonyl group; arylsulfonyl groups which may be substituted such as p-toluenesulfonyl group, p-methoxybenzenesulfonyl group, and p-fluorobenzenesulfonyl group; aralkyl groups such as benzyl group, naphthylmethyl group, pyridylmethyl group, furfuryl group, and triphenylmethyl group; nitro group; cyano group; azido group; sulfamoyl group; oxo group; hydroxyimino group; alkoxyimino groups such as methoxyimino group, ethoxyimino group, n-propoxyimino group, and isopropoxyimino group; ethylenedioxy group and the like.

In the specification, some examples are given with respect to the substituting/binding position of an aryl group of "the aryl group", "the homocyclic or heterocyclic ring", "the acyl group", "the aryloxy group", "the amino group which may be substituted", "the arylsulfonyl group", and "the aralkyl group". However, the aforementioned groups may substitute/bind at any position unless a substituting/binding position is specifically limited.

The compounds represented by the aforementioned general formulas (I) and (II) of the present invention can be converted into salts, preferably, pharmacologically acceptable salts, if desired; or free bases can be generated from the resulting salts.

Examples of the salts, preferably the pharmacologically acceptable salts of the compounds represented by the aforementioned general formulas (I) and (II) of the present invention include acid-addition salts, for example, salts with mineral acids such as hydrochloric acid, hydrobromic acid, hydroiodic acid, sulfuric acid, nitric acid, and phosphoric acid; and salts with organic acids such as formic acid, acetic acid, propionic acid, butyric acid, isobutyric acid, valeric

acid, isovaleric acid, pivalic acid, trifluoroacetic acid, acrylic acid, oleic acid, maleic acid, fumaric acid, citric acid, oxalic acid, succinic acid, tartaric acid, malic acid, malonic acid, lactic acid, glutaric acid, sebacic acid, gluconic acid, lauric acid, myristic acid, stearic acid, undecanoic acid, mandelic acid, methanesulfonic acid, ethanesulfonic acid, benzenesulfonic acid, benzoic acid, phthalic acid, terephthalic acid, cinnamic acid, p-toluenesulfonic acid, nicotinic acid, picric acid, adipic acid, aspartic acid, glutamic acid, 10-camphorsulfonic acid, enantiomers thereof and the like.

Among the compounds represented by the aforementioned general formulas (I) and (II) of the present invention, optical isomers or diastereomers may exist for compounds having one or more asymmetric carbons. These optical isomers and mixtures thereof, racemates or salts thereof also fall within the scope of the present invention.

The compounds represented by the aforementioned general formulas (I) and (II) or the salts thereof according to the present invention can exist as any crystalline form depending on manufacturing conditions, or exist as any hydrate or solvate. These crystalline forms, hydrates or solvates, and mixtures thereof also fall within the scope of the present invention.

Preferred compounds of the present invention include, for example, the following compounds and salts thereof; however, the present invention is not limited to these examples:

(1) 1-[2-(4-Piperidyl)ethyl]-4-trifluoromethyl-1H-imidazo[4,5-c]quinoline
(2) 2-Phenyl-1-[2-(4-piperidyl)ethyl]-4-trifluoromethyl-1H-imidazo[4,5-c]quinoline
(3) 8-Chloro-2-phenyl-1-[2-(4-piperidyl)ethyl]-4-trifluoromethyl-1H-imidazo[4,5-c]quinoline
(4) 8-Methyl-2-phenyl-1-[2-(4-piperidyl)ethyl]-4-trifluoromethyl-1H-imidazo[4,5-c]quinoline
(5) 8-Methoxy-2-phenyl-1-[2-(4-piperidyl)ethyl]-4-trifluoromethyl-1H-imidazo[4,5-c]quinoline
(6) 1-[2-(4-Piperidyl)ethyl]-2,4-ditrifluoromethyl-1H-imidazo[4,5-c]quinoline
(7) 2-Cyclopentyl-1-[2-(4-piperidyl)ethyl]-4-trifluoromethyl-1H-imidazo[4,5-c]quinoline
(8) 2-Cyclohexyl-1-[2-(4-piperidyl)ethyl]-4-trifluoromethyl-1H-imidazo[4,5-c]quinoline
(9) 2-tert-Butyl-1-[2-(4-piperidyl)ethyl]-4-trifluoromethyl-1H-imidazo[4,5-c]quinoline
(10) 2-(4-Methylphenyl)-1-[2-(4-piperidyl)ethyl]-4-trifluoromethyl-1H-imidazo[4,5-c]quinoline
(11) 2-(4-Methoxyphenyl)-1-[2-(4-piperidyl)ethyl]-4-trifluoromethyl-1H-imidazo[4,5-c]quinoline
(12) 2-(4-Fluorophenyl)-1-[2-(4-piperidyl)ethyl]-4-trifluoromethyl-1H-imidazo[4,5-c]quinoline
(13) 1-[2-(4-Piperidyl)ethyl]-4-trifluoromethyl-2-(4-trifluoromethylphenyl)-1Himidazo[4,5-c]quinoline
(14) 2-(4-Iodophenyl)-1-[2-(4-piperidyl)ethyl]-4-trifluoromethyl-1H-imidazo[4,5-c]quinoline
(15) 1-[2-(4-Piperidyl)ethyl]-2-(2-pyrrolyl)-4-trifluoromethyl-1H-imidazo[4,5-c]quinoline
(16) 2-(2-1H-Imidazolyl)-1-[2-(4-piperidyl)ethyl]-4-trifluoromethyl-1H-imidazo[4,5-c]quinoline
(17) 1-[2-(4-Piperidyl)ethyl]-2-(2-thiazolyl)-4-trifluoromethyl-1H-imidazo[4,5-c]quinoline
(18) 1-[2-(4-Piperidyl)ethyl]-2-(2-thienyl)-4-trifluoromethyl-1H-imidazo[4,5-c]quinoline
(19) 2-(5-Methyl-2-thienyl)-1-[2-(4-piperidyl)ethyl]-4-trifluoromethyl-1H-imidazo[4,5-c]quinoline
(20) 2-(3-Methyl-2-thienyl)-1-[2-(4-piperidyl)ethyl]-4-trifluoromethyl-1H-imidazo[4,5-c]quinoline
(21) 2-(2-Furyl)-1-[2-(4-piperidyl)ethyl]-4-trifluoromethyl-1H-imidazo[4,5-c]quinoline
(22) 7-Chloro-2-phenyl-1-[2-(4-piperidyl)ethyl]-4-trifluoromethyl-1H-imidazo[4,5-c]quinoline
(23) 7-Chloro-1-[2-(4-piperidyl)ethyl]-2-(2-pyrrolyl)-4-trifluoromethyl-1H-imidazo[4,5-c]quinoline
(24) 7-Chloro-1-[2-(4-piperidyl)ethyl]-2-(2-thiazolyl)-4-trifluoromethyl-1H-imidazo[4,5-c]quinoline
(25) 7-Chloro-2-(2-1H-imidazolyl)-1-[2-(4-piperidyl)ethyl]-4-trifluoromethyl-1Himidazo[4,5-c]quinoline
(26) 7-Chloro-2-(2-furyl)-1-[2-(4-piperidyl)ethyl]-4-trifluoromethyl-1H-imidazo[4,5-c]quinoline
(27) 7-Chloro-1-[2-(4-piperidyl)ethyl]-2-(2-thienyl)-4-trifluoromethyl-1H-imidazo[4,5-c]quinoline
(28) 7-Chloro-2-cyclopentyl-1-[2-(4-piperidyl)ethyl]-4-trifluoromethyl-1H-imidazo[4,5-c]quinoline
(29) 7-Chloro-2-cyclohexyl-1-[2-(4-piperidyl)ethyl]-4-trifluoromethyl-1H-imidazo[4,5-c]quinoline
(30) 7-Fluoro-2-phenyl-1-[2-(4-piperidyl)ethyl]-4-trifluoromethyl-1H-imidazo[4,5-c]quinoline
(31) 7-Fluoro-1-[2-(4-piperidyl)ethyl]-2-(2-pyrrolyl)-4-trifluoromethyl-1H-imidazo[4,5-c]quinoline
(32) 7-Fluoro-1-[2-(4-piperidyl)ethyl]-2-(2-thiazolyl)-4-trifluoromethyl-1H-imidazo[4,5-c]quinoline
(33) 7-Fluoro-2-(2-1H-imidazolyl)-1-[2-(4-piperidyl)ethyl]-4-trifluoromethyl-1Himidazo[4,5-c]quinoline
(34) 7-Fluoro-2-(2-furyl)-1-[2-(4-piperidyl)ethyl]-4-trifluoromethyl-1H-imidazo[4,5-c]quinoline
(35) 7-Fluoro-1-[2-(4-piperidyl)ethyl]-2-(2-thienyl)-4-trifluoromethyl-1H-imidazo[4,5-c]quinoline
(36) 2-Cyclopentyl-7-fluoro-1-[2-(4-piperidyl)ethyl]-4-trifluoromethyl-1H-imidazo[4,5-c]quinoline
(37) 2-Cyclohexyl-7-fluoro-1-[2-(4-piperidyl)ethyl]-4-trifluoromethyl-1H-imidazo[4,5-c]quinoline
(38) 7-Methyl-2-phenyl-1-[2-(4-piperidyl)ethyl]-4-trifluoromethyl-1H-imidazo[4,5-c]quinoline
(39) 7-Methyl-1-[2-(4-piperidyl)ethyl]-2-(2-pyrrolyl)-4-trifluoromethyl-1H-imidazo[4,5-c]quinoline
(40) 7-Methyl-1-[2-(4-piperidyl)ethyl]-2-(2-thiazolyl)-4-trifluoromethyl-1H-imidazo[4,5-c]quinoline
(41) 2-(2-1H-Imidazolyl)-7-methyl-1-[2-(4-piperidyl)ethyl]-4-trifluoromethyl-1Himidazo[4,5-c]quinoline
(42) 2-(2-Furyl)-7-methyl-1-[2-(4-piperidyl)ethyl]-4-trifluoromethyl-1H-imidazo[4,5-c]quinoline
(43) 7-Methyl-1-[2-(4-piperidyl)ethyl]-2-(2-thienyl)-4-trifluoromethyl-1H-imidazo[4,5-c]quinoline

(44) 2-Cyclopentyl-7-methyl-1-[2-(4-piperidyl)ethyl]-4-trifluoromethyl-1H-imidazo[4,5-c]quinoline
(45) 2-Cyclohexyl-7-methyl-1-[2-(4-piperidyl)ethyl]-4-trifluoromethyl-1H-imidazo[4,5-c]quinoline
(46) 6-Methyl-2-phenyl-1-[2-(4-piperidyl)ethyl]-4-trifluoromethyl-1H-imidazo[4,5-c]quinoline
(47) 6-Methyl-1-[2-(4-piperidyl)ethyl]-2-(2-pyrrolyl)-4-trifluoromethyl-1H-imidazo[4,5-c]quinoline
(48) 6-Methyl-1-[2-(4-piperidyl)ethyl]-2-(2-thiazolyl)-4-trifluoromethyl-1H-imidazo[4,5-c]quinoline
(49) 2-(2-1H-Imidazolyl)-6-methyl-1-[2-(4-piperidyl)ethyl]-4-trifluoromethyl-1H-imidazo[4,5-c]quinoline
(50) 2-(2-Furyl)-6-methyl-1-[2-(4-piperidyl)ethyl]-4-trifluoromethyl-1H-imidazo[4,5-c]quinoline
(51) 6-Methyl-1-[2-(4-piperidyl)ethyl]-2-(2-thienyl)-4-trifluoromethyl-1H-imidazo[4,5-c]quinoline
(52) 2-Cyclopentyl-6-methyl-1-[2-(4-piperidyl)ethyl]-4-trifluoromethyl-1H-imidazo[4,5-c]quinoline
(53) 2-Cyclohexyl-6-methyl-1-[2-(4-piperidyl)ethyl]-4-trifluoromethyl-1H-imidazo[4,5-c]quinoline
(54) 7-Methoxy-2-phenyl-1-[2-(4-piperidyl)ethyl]-4-trifluoromethyl-1H-imidazo[4,5-c]quinoline
(55) 2-Phenyl-1-[2-(4-piperidyl)ethyl]-4,7-ditrifluoromethyl-1H-imidazo[4,5-c]quinoline
(56) 7-Chloro-2-phenyl-1-[2-(1-piperazinyl)ethyl]-4-trifluoromethyl-1H-imidazo[4,5-c]quinoline
(57) 7-Chloro-1-[2-(1-piperazinyl)ethyl]-2-(2-pyrrolyl)-4-trifluoromethyl-1H-imidazo[4,5-c]quinoline
(58) 7-Chloro-1-[2-(1-piperazinyl)ethyl]-2-(2-thiazolyl)-4-trifluoromethyl-1H-imidazo[4,5-c]quinoline
(59) 7-Chloro-2-(2-1H-imidazolyl)-1-[2-(1-piperazinyl)ethyl]-4-trifluoromethyl-1H-imidazo[4,5-c]quinoline
(60) 7-Chloro-2-(2-furyl)-1-[2-(1-piperazinyl)ethyl]-4-trifluoromethyl-1H-imidazo[4,5-c]quinoline
(61) 7-Chloro-1-[2-(1-piperazinyl)ethyl]-2-(2-thienyl)-4-trifluoromethyl-1H-imidazo[4,5-c]quinoline
(62) 7-Chloro-2-cyclopentyl-1-[2-(1-piperazinyl)ethyl]-4-trifluoromethyl-1H-imidazo[4,5-c]quinoline
(63) 7-Chloro-2-cyclohexyl-1-[2-(1-piperazinyl)ethyl]-4-trifluoromethyl-1H-imidazo[4,5-c]quinoline
(64) 7-Fluoro-2-phenyl-1-[2-(1-piperazinyl)ethyl]-4-trifluoromethyl-1H-imidazo[4,5-c]quinoline
(65) 7-Fluoro-1-[2-(1-piperazinyl)ethyl]-2-(2-pyrrolyl)-4-trifluoromethyl-1H-imidazo[4,5-c]quinoline
(66) 7-Fluoro-1-[2-(1-piperazinyl)ethyl]-2-(2-thiazolyl)-4-trifluoromethyl-1H-imidazo[4,5-c]quinoline
(67) 7-Fluoro-2-(2-1H-imidazolyl)-1-(2-(1-piperazinyl)ethyl]-4-trifluoromethyl-1Himidazo[4,5-c]quinoline
(68) 7-Fluoro-2-(2-furyl)-1-[2-(1-piperazinyl)ethyl]-4-trifluoromethyl-1H-imidazo[4,5-c]quinoline
(69) 7-Fluoro-1-[2-(1-piperazinyl)ethyl]-2-(2-thienyl)-4-trifluoromethyl-1H-imidazo[4,5-c]quinoline
(70) 2-Cyclopentyl-7-fluoro-1-[2-(1-piperazinyl)ethyl]-4-trifluoromethyl-1H-imidazo[4,5-c]quinoline
(71) 2-Cyclohexyl-7-fluoro-1-[2-(1-piperazinyl)ethyl]-4-trifluoromethyl-1H-imidazo[4,5-c]quinoline
(72) 7-Methyl-2-phenyl-1-[2-(1-piperazinyl)ethyl]-4-trifluoromethyl-1H-imidazo[4,5-c]quinoline
(73) 7-Methyl-1-[2-(1-piperazinyl)ethyl]-2-(2-pyrrolyl)-4-trifluoromethyl-1H-imidazo[4,5-c]quinoline
(74) 7-Methyl-1-[2-(1-piperazinyl)ethyl]-2-(2-thiazolyl)-4-trifluoromethyl-1H-imidazo[4,5-c]quinoline
(75) 2-(2-1H-Imidazolyl)-7-methyl-1-[2-(1-piperazinyl)ethyl]-4-trifluoromethyl-1H-imidazo[4,5-c]quinoline
(76) 2-(2-Furyl)-7-methyl-1-[2-(1-piperazinyl)ethyl]-4-trifluoromethyl-1H-imidazo[4,5-c]quinoline
(77) 7-Methyl-1-[2-(1-piperazinyl)ethyl]-2-(2-thienyl)-4-trifluoromethyl-1H-imidazo[4,5-c]quinoline
(78) 2-Cyclopentyl-7-methyl-1-[2-(1-piperazinyl)ethyl]-4-trifluoromethyl-1H-imidazo[4,5-c]quinoline
(79) 2-Cyclohexyl-7-methyl-1-[2-(1-piperazinyl)ethyl]-4-trifluoromethyl-1H-imidazo[4,5-c]quinoline
(80) 6-Methyl-2-phenyl-1-[2-(1-piperazinyl)ethyl]-4-trifluoromethyl-1H-imidazo[4,5-c]quinoline
(81) 6-Methyl-1-[2-(1-piperazinyl)ethyl]-2-(2-pyrrolyl)-4-trifluoromethyl-1H-imidazo[4,5-c]quinoline
(82) 6-Methyl-1-[2-(1-piperazinyl)ethyl]-2-(2-thiazolyl)-4-trifluoromethyl-1H-imidazo[4,5-c]quinoline
(83) 2-(2-1H-Imidazolyl)-6-methyl-1-[2-(1-piperazinyl)ethyl]-4-trifluoromethyl-1Himidazo[4,5-c]quinoline
(84)2-(2-Furyl)-6-methyl-1-[2-(1-piperazinyl)ethyl]-4-trifluoromethyl-1H-imidazo[4,5-c]quinoline
(85) 6-Methyl-1-[2-(1-piperazinyl)ethyl]-2-(2-thienyl)-4-trifluoromethyl-1H-imidazo[4,5-c]quinoline
(86) 2-Cyclopentyl-6-methyl-1-[2-(1-piperazinyl)ethyl]-4-trifluoromethyl-1H-imidazo[4,5-c]quinoline
(87) 2-Cyclohexyl-6-methyl-1-[2-(1-piperazinyl)ethyl]-4-trifluoromethyl-1H-imidazo[4,5-c]quinoline
(88) 7-Chloro-1-[2-(2-morpholinyl)ethyl]-2-phenyl-4-trifluoromethyl-1H-imidazo[4,5-c]quinoline
(89) 7-Chloro-1-[2-(2-morpholinyl)ethyl]-2-(2-pyrrolyl)-4-trifluoromethyl-1H-imidazo[4,5-c]quinoline
(90) 7-Chloro-1-[2-(2-morpholinyl)ethyl]-2-(2-thiazolyl)-4-trifluoromethyl-1H-imidazo[4,5-c]quinoline
(91) 7-Chloro-2-(2-1H-imidazolyl)-1-[2-(2-morpholinyl)ethyl]-4-trifluoromethyl-1H-imidazo[4,5-c]quinoline
(92) 7-Chloro-2-(2-furyl)-1-[2-(2-morpholinyl)ethyl]-4-trifluoromethyl-1H-imidazo[4,5-c]quinoline
(93) 7-Chloro-1-[2-(2-morpholinyl)ethyl]-2-(2-thienyl)-4-trifluoromethyl-1H-imidazo[4,5-c]quinoline
(94) 7-Chloro-2-cyclopentyl-1-[2-(2-morpholinyl)ethyl]-4-trifluoromethyl-1H-imidazo[4,5-c]quinoline
(95) 7-Chloro-2-cyclohexyl-1-[2-(2-morpholinyl)ethyl]-4-trifluoromethyl-1H-imidazo[4,5-clquinoline
(96) 7-Fluoro-1-[2-(2-morpholinyl)ethyl]-2-phenyl-4-trifluoromethyl-1H-imidazo[4,5-c]quinoline
(97) 7-Fluoro-1-[2-(2-morpholinyl)ethyl]-2-(2-pyrrolyl)-4-trifluoromethyl-1H-imidazo[4,5-c]quinoline
(98) 7-Fluoro-1-[2-(2-morpholinyl)ethyl]-2-(2-thiazolyl)-4-trifluoromethyl-1H-imidazo[4,5-c]quinoline
(99) 7-Fluoro-2-(2-1H-imidazolyl)-1-[2-(2-morpholinyl)ethyl]-4-trifluoromethyl-1Himidazo[4,5-c]quinoline
(100) 7-Fluoro-2-(2-furyl)-1-[2-(2-morpholinyl)ethyl]-4-trifluoromethyl-1H-imidazo[4,5-c]quinoline
(101) 7-Fluoro-1-[2-(2-morpholinyl)ethyl]-2-(2-thienyl)-4-trifluoromethyl-1H-imidazo[4,5-c]quinoline

(102) 2-Cyclopentyl-7-fluoro-1-[2-(2-morpholinyl)ethyl]-4-trifluoromethyl-1H-imidazo[4,5-c]quinoline
(103) 2-Cyclohexyl-7-fluoro-1-[2-(2-morpholinyl)ethyl]-4-trifluoromethyl-1H-imidazo[4,5-c]quinoline
(104) 7-Methyl-1-[2-(2-morpholinyl)ethyl]-2-phenyl-4-trifluoromethyl-1H-imidazo[4,5-c]quinoline
(105) 7-Methyl-1-[2-(2-morpholinyl)ethyl]-2-(2-pyrrolyl)-4-trifluoromethyl-1H-imidazo[4,5-c]quinoline
(106) 7-Methyl-1-[2-(2-morpholinyl)ethyl]-2-(2-thiazolyl)-4-trifluoromethyl-1H-imidazo[4,5-c]quinoline
(107) 2-(2-1H-Imidazolyl)-7-methyl-1-[2-(2-morpholinyl)ethyl]-4-trifluoromethyl-1H-imidazo[4,5-c]quinoline
(108) 2-(2-Furyl)-7-methyl-1-[2-(2-morpholinyl)ethyl]-4-trifluoromethyl-1H-imidazo[4,5-c]quinoline
(109) 7-Methyl-1-[2-(2-morpholinyl)ethyl]-2-(2-thienyl)-4-trifluoromethyl-1H-imidazo[4,5-c]quinoline
(110) 2-Cyclopentyl-7-methyl-1-[2-(2-morpholinyl)ethyl]-4-trifluoromethyl-1Himidazo[4,5-c]quinoline
(111) 2-Cyclohexyl-7-methyl-1-[2-(2-morpholinyl)ethyl]-4-trifluoromethyl-1H-imidazo[4,5-c]quinoline
(112) 6-Methyl-1-[2-(2-morpholinyl)ethyl]-2-phenyl-4-trifluoromethyl-1H-imidazo[4,5-c]quinoline
(113) 6-Methyl-1-[2-(2-morpholinyl)ethyl]-2-(2-pyrrolyl)-4-trifluoromethyl-1H-imidazo[4,5-c]quinoline
(114) 6-Methyl-1-[2-(2-morpholinyl)ethyl]-2-(2-thiazolyl)-4-trifluoromethyl-1H-imidazo[4,5-c]quinoline
(115) 2-(2-1H-Imidazolyl)-6-methyl-1-[2-(2-morpholinyl)ethyl]-4-trifluoromethyl-1H-imidazo[4,5-c]quinoline
(116) 2-(2-Furyl)-6-methyl-1-[2-(2-morpholinyl)ethyl]-4-trifluoromethyl-1H-imidazo[4,5-c]quinoline
(117) 6-Methyl-1-[2-(2-morpholinyl)ethyl]-2-(2-thienyl)-4-trifluoromethyl-1H-imidazo[4,5-c]quinoline
(118) 2-Cyclopentyl-6-methyl-1-[2-(2-morpholinyl)ethyl]-4-trifluoromethyl-1H-imidazo[4,5-c]quinoline
(119) 2-Cyclohexyl-6-methyl-1-[2-(2-morpholinyl)ethyl]-4-trifluoromethyl-1H-imidazo[4,5-c]quinoline
(120) 4-Cyclopropyl-2-phenyl-1-[2-(4-piperidyl)ethyl]-1H-imidazo[4,5-c]quinoline
(121) 4-Cyclopropyl-1-[2-(4-piperidyl)ethyl]-2-(2-pyrrolyl)-1H-imidazo[4,5-c]quinoline
(122) 2,4-biphenyl-1-[2-(4-piperidyl)ethyl]-1H-imidazo[4,5-c]quinoline
(123) 4-Phenyl-1-[2-(4-piperidyl)ethyl]-2-(2-pyrrolyl)-1H-imidazo[4,5-c]quinoline
(124) 4-(2-Furyl)-2-phenyl-1-[2-(4-piperidyl)ethyl]-1H-imidazo[4,5-c]quinoline
(125) 2-Phenyl-1-[2-(4-piperidyl)ethyl]-4-(2-thienyl)-1H-imidazo[4,5-c]quinoline
(126) 4-Cyano-2-phenyl-1-[2-(4-piperidyl)ethyl]-1H-imidazo[4,5-c]quinoline
(127) 4-Mercapto-2-phenyl-1-[2-(4-piperidyl)ethyl]-1H-imidazo[4,5-c]quinoline
(128) 2-Phenyl-1-[2-(4-piperidyl)ethyl]-1H-imidazo[4,5-c]quinoline-4-carboxylic acid
(129) 2-Phenyl-1-[2-(4-piperidyl)ethyl]-1H-imidazo[4,5-c]quinoline-4-carboxamide
(130)2-Phenyl-1-[2-(1-piperazinyl)ethyl]-4-trifluoromethyl-1H-imidazo[4,5-c]quinoline
(131) 1-[2-(1-Piperazinyl)ethyl]-2-(2-pyrrolyl)-4-trifluoromethyl-1H-imidazo[4,5-c]quinoline
(132) 2-Phenyl-1-[2-(3-piperidyl)ethyl]-4-trifluoromethyl-1H-imidazo[4,5-c]quinoline
(133) 1-[2-(3-Piperidyl)ethyl]-2-(2-pyrrolyl)-4-trifluoromethyl-1H-imidazo[4,5-c]quinoline
(134) 1-[2-(2-Morpholinyl)ethyl]-2-phenyl-4-trifluoromethyl-1H-imidazo[4,5-c]quinoline
(135) 1-[2-(2-Morpholinyl)ethyl]-2-(2-pyrrolyl)-4-trifluoromethyl-1H-imidazo[4,5-c]quinoline
(136) 2-Ethoxymethyl-1-[2-(2-thiomorpholinyl)ethyl]-4-trifluoromethyl-1H-imidazo[4,5-c]quinoline
(137) 1-[2-(8-Azabicyclo[3.2.1]octan-3-yl)ethyl]-2-phenyl-4-trifluoromethyl-1H-imidazo[4,5-c]quinoline
(138) 1-[2-(8-Azabicyclo[3.2.1]octan-3-yl)ethyl]-2-(2-pyrrolyl)-4-trifluoromethyl-1H-imidazo[4,5-c]quinoline
(139) 1-(2-Aminoethyl)-2-phenyl-4-trifluoromethyl-1H-imidazo[4,5-c]quinoline
(140) 1-(2-Aminoethyl)-2-(2-pyrrolyl)-4-trifluoromethyl-1H-imidazo[4,5-c]quinoline
(141) 1-(2-Dimethylaminoethyl)-2-phenyl-4-trifluoromethyl-1H-imidazo[4,5-c]quinoline
(142) 1-(2-Dimethylaminoethyl)-2-(2-pyrrolyl)-4-trifluoromethyl-1H-imidazo[4,5-c]quinoline
(143) 2-Phenyl-1-[2-(3-pyrrolidinyl)ethyl]-4-trifluoromethyl-1H-imidazo[4,5-c]quinoline
(144) 1-[2-(3-Azetidinyl)ethyl]-2-(2-pyrrolyl)-4-trifluoromethyl-1H-imidazo[4,5-c]quinoline
(145) 6,7,8,9-Tetrahydro-2-phenyl-1-[2-(4-piperidyl)ethyl]-4-trifluoromethyl-1H-imidazo[4,5-c]quinoline
(146) 6,7-Dihydro-2-phenyl-1-[2-(4-piperidyl)ethyl]-4-trifluoromethyl-1H-imidazo[5,4-d]cyclopenta[b]pyridine
(147) 6,7-Dihydro-1-[2-(4-piperidyl)ethyl]-2-(2-pyrrolyl)-4-trifluoromethyl-1H-imidazo[5,4-d]cyclopenta[b]pyridine
(148) 6,7-Dihydro-1-[2-(4-piperidyl)ethyl]-2-(2-thiazolyl)-4-trifluoromethyl-1H-imidazo[5,4-d]cyclopenta[b]pyridine
(149) 6,7-Dihydro-2-(2-1H-imidazolyl)-1-[2-(4-piperidyl)ethyl]-4-trifluoromethyl-1H-imidazo[5,4-d]cyclopenta[b]pyridine
(150) 2-(2-Furyl)-6,7-dihydro-1-[2-(4-piperidyl)ethyl]-4-trifluoromethyl-1H-imidazo[5,4-d]cyclopenta[b]pyridine
(151) 6,7-Dihydro-1-[2-(4-piperidyl)ethyl]-2-(2-thienyl)-4-trifluoromethyl-1H-imidazo[5,4-d]cyclopenta[b]pyridine
(152) 2-Cyclopentyl-6,7-dihydro-1-[2-(4-piperidyl)ethyl]-4-trifluoromethyl-1H-imidazo[5,4-d]cyclopenta[b]pyridine
(153) 2-Cyclohexyl-6,7-dihydro-1-[2-(4-piperidyl)ethyl]-4-trifluoromethyl-1H-imidazo[5,4-d]cyclopenta[b]pyridine
(154) 2-Phenyl-1-[2-(4-piperidyl)ethyl]-4-trifluoromethyl-1H-imidazo[5,4-d]thieno[3,2-b]pyridine
(155) 2-Phenyl-1-[2-(4-piperidyl)ethyl]-4-trifluoromethyl-1H-imidazo[4,5-c][1,5]naphthyridine
(156) 2-Phenyl-1-(4-piperidyl)-4-trifluoromethyl-1H-imidazo[4,5-c]quinoline
(157) 2-Phenyl-1-[3-(4-piperidyl)propyl]-4-trifluoromethyl-1H-imidazo[4,5-c]quinoline
(158) 1-[2-(n-Butylamino)ethyl]-4-methyl-2-phenyl-1H-imidazo[4,5-c]quinoline

(159) 1-[2-(Benzylamino)ethyl]-4-methyl-2-phenyl-1H-imidazo[4,5-c]quinoline
(160) 4-Methyl-2-phenyl-1-[2-(triphenylmethylamino)ethyl]-1H-imidazo[4,5-c]quinoline
(161) Benzyl N-[2-(4-methyl-2-phenyl-1H-imidazo[4,5-c]quinolin-1-yl)ethyl]carbamate
(162) N-[2-(4-Methyl-2-phenyl-1H-imidazo[4,5-c]quinolin-1-yl)ethyl]acetamide
(163) N-Methyl-N'-[2-(4-methyl-2-phenyl-1H-imidazo[4,5-c]quinolin-1-yl)ethyl]thiourea
(164) N-[2-(4-Methyl-2-phenyl-1H-imidazo[4,5-c]quinolin-1-yl)ethyl]methanesulfonamide
(165) N-[2-(4-Methyl-2-phenyl-1H-imidazo[4,5-c]quinolin-1-yl)ethyl] p-toluenesulfonamide
(166) 1-(2-Guanidinoethyl)-4-methyl-2-phenyl-1H-imidazo[4,5-c]quinoline
(167) 2-Methylamino-N-[2-(2-phenyl-4-trifluoromethyl-1H-imidazo[4,5-c]quinolin-1-yl)ethyl]acetamide
(168) 2-Methylamino-N-[2-[2-(2-pyrrolyl)-4-trifluoromethyl-1H-imidazo[4,5-c]quinolin-1-yl]ethyl]acetamide
(169) 2-Dimethylamino-N-[2-(2-phenyl-4-trifluoromethyl-1H-imidazo[4,5-c]quinolin-1-yl)ethyl]acetamide
(170) 2-Dimethylamino-N-[2-[2-(2-pyrrolyl)-4-trifluoromethyl-1H-imidazo[4,5-c]quinolin-1-yl]ethyl]acetamide
(171) 2-Amino-N-[2-(2-phenyl-4-trifluoromethyl-1H-imidazo[4,5-c]quinolin-1-yl)ethyl]acetamide
(172) 2-Amino-N-[2-[2-(2-pyrrolyl)-4-trifluoromethyl-1H-imidazo[4,5-c]quinolin-1-yl]ethyl]acetamide
(173) 1-Acetyl-4-[2-(2-phenyl-4-trifluoromethyl-1H-imidazo[4,5-c]quinolin-1-yl)ethyl]piperidine
(174) 1-[2-(1-Benzyl-4-piperidyl)ethyl]-2-phenyl-4-trifluoromethyl-1H-imidazo[4,5-c]quinoline
(175) 7-Chloro-2-(2-hydroxyphenyl)-1-[2-(4-piperidyl)ethyl]-4-trifluoromethyl-1H-imidazo[4,5-c]quinoline
(176) 7-Chloro-2-(2-methoxyphenyl)-1-[2-(4-piperidyl)ethyl]-4-trifluoromethyl-1H-imidazo[4,5-c]quinoline
(177) 7-Chloro-2-(3-hydroxyphenyl)-1-[2-(4-piperidyl)ethyl]-4-trifluoromethyl-1H-imidazo[4,5-c]quinoline
(178) 7-Chloro-2-(3-methoxyphenyl)-1-[2-(4-piperidyl)ethyl]-4-trifluoromethyl-1H-imidazo[4,5-c]quinoline
(179) 7-Chloro-2-(4-hydroxyphenyl)-1-[2-(4-piperidyl)ethyl]-4-trifluoromethyl-1H-imidazo[4,5-c]quinoline
(180) 7-Chloro-2-(4-methoxyphenyl)-1-[2-(4-piperidyl)ethyl]-4-trifluoromethyl-1H-imidazo[4,5-c]quinoline
(181) 2-(2-Hydroxyphenyl)-7-methyl-1-[2-(4-piperidyl)ethyl]-4-trifluoromethyl-1H-imidazo[4,5-c]quinoline
(182) 2-(2-Methoxyphenyl)-7-methyl-1-[2-(4-piperidyl)ethyl]-4-trifluoromethyl-1H-imidazo[4,5-c]quinoline
(183) 2-(3-Hydroxyphenyl)-7-methyl-1-[2-(4-piperidyl)ethyl]-4-trifluoromethyl-1H-imidazo[4,5-c]quinoline
(184) 2-(3-Methoxyphenyl)-7-methyl-1-[2-(4-piperidyl)ethyl]-4-trifluoromethyl-1H-imidazo[4,5-c]quinoline
(185) 2-(4-Hydroxyphenyl)-7-methyl-1-[2-(4-piperidyl)ethyl]-4-trifluoromethyl-1H-imidazo[4,5-c]quinoline
(186) 2-(4-Methoxyphenyl)-7-methyl-1-[2-(4-piperidyl)ethyl]-4-trifluoromethyl-1H-imidazo[4,5-c]quinoline

The novel 1H-imidazopyridine derivatives represented by the aforementioned general formula (I) or (II) according to the present invention can be prepared by, for example, various methods such as exemplified below; however, the preparation methods of the compounds of the present invention are not limited thereto. In the following preparation methods, specific explanations for the compounds represented by the aforementioned general formula (I) will be given, and it is obvious that these preparation methods include the preparation methods of the compounds represented by the aforementioned general formula (II).

As the first synthetic method of the compounds of the present invention, the following preparation can be carried out in accordance with the method disclosed in Japanese Patent Unexamined Publication (KOKAI) No. Hei 3-206078/1991 or Tetrahedron, Vol. 51, p. 5813 (1995):

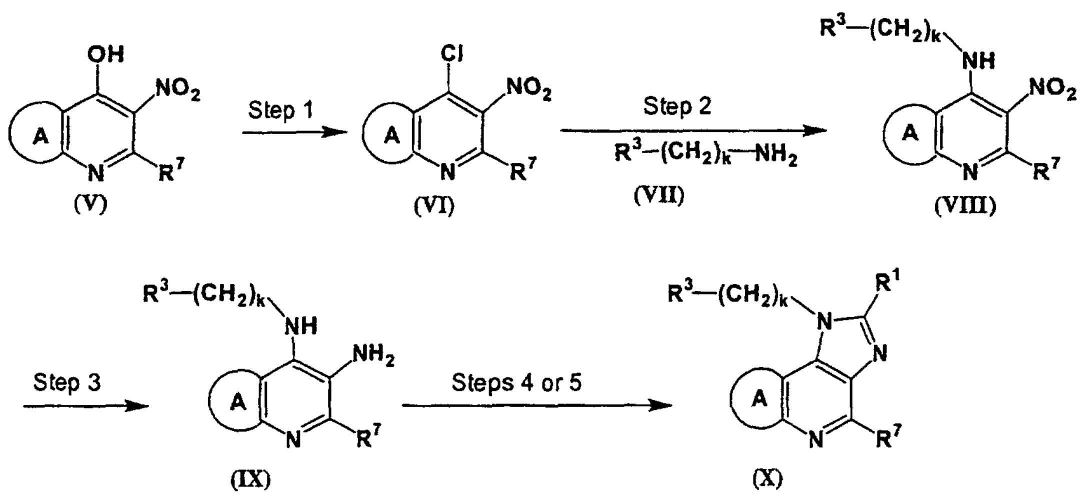

wherein $R^7$ represents a cycloalkyl group which may be substituted, an alkyl group which may be substituted, or an aryl group which may be substituted;, and $R^1$, $R^3$, k, and ring A have the same meanings as those defined above.

In Step 1, the compound of the general formula (VI) can be obtained by allowing the compound represented by the general formula (V) to react with a chlorinating agent, for example, phosphorus ogychloride, thionyl chloride, phosgene, oxalyl chloride, phosphorus pentachloride or the like, in the presence or absence of a solvent such as toluene and N,N-dimethylformamide at a temperature ranging from 0°C to 200°C.

In Step 2, the compound of the general formula (VIII) can be obtained by reacting the amine represented by the general formula (VII) with the compound of the general formula (VI) in a solvent such as N,N-dimethylformamide and toluene in the presence or absence of a base such as triethylamine and potassium carbonate at a temperature ranging from -10°C to the reflux temperature of a solvent.

In Step 3, the compound of the general formula (IX) can be obtained by reducing the nitro group of the compound of the general formula (VIII) according to an ordinarily-used reducing method, for example, catalytic reduction using a metal catalyst such as platinum, Raney nickel, and palladium/carbon; reduction using nickel chloride and sodium borohydride; reduction using iron powder and hydrochloric acid and the like.

In Step 4, the compound of the general formula (X) can be obtained by reacting the compound of the general formula (IX) with a compound represented by the following general formula (XI), (XII), (XIII), or (XIV):

$$R^1C(OR)_3 \quad (XI)$$

$$R^1COX \quad (XII)$$

$$(R^1CO)_2O \quad (XIII)$$

$$R^1CO_2H \quad (XIV)$$

wherein R represents a lower alkyl group; X represents a halogen atom; $R^1$ has the same meaning as that defined above,

in the presence or absence of a basic catalyst such as triethylamine, N,N-diisopropylethylamine, pyridine, sodium carbonate, and potassium carbonate, or an acid catalyst such as hydrochloric acid, sulfuric acid, and p-toluenesulfonic acid, in the presence or absence of a solvent such as N,N-dimethylformamide, 1,2-dichloroethane, tetrahydrofuran, acetonitrile, xylene, and toluene, at a temperature ranging from 0°C to 200°C.

As an alternate step for Step 4, the compound of the general formula (X) can be obtained in Step 5 by reacting the compound of the general formula (IX) with a compound represented by the following general formula (XV):

$$R^1CHO \quad (XV)$$

wherein $R^1$ has the same meanings as that defined above,

in the presence of 2,3-dichloro-5,6-dicyano-1,4-benzoquinone in a solvent such as acetonitrile, 1,4-dioxane, tetrahydrofuran, 1,2-dichloroethane, and toluene at a temperature ranging from 0°C to the reflux temperature of the solvent.

According to the second synthetic method of the compounds of the present invention, the compound of the following general formula (XVII):

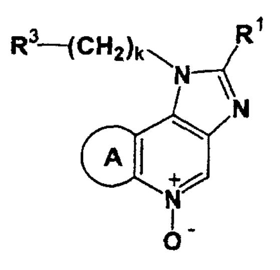

(XVII)

wherein $R^1$, $R^3$, k, and ring A have the same meanings as those defined above,
can be obtained by reacting the compound of the following general formula (XVI) which can be synthesized by the method disclosed in Japanese Patent Unexamined Publication(KOKAI) No. Sho 60-123488/1985:

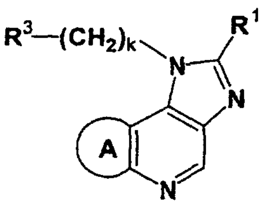

(XVI)

wherein $R^1$, $R^3$, k, and ring A have the same meanings as those defined above, with an oxidizing agent such as hydrogen peroxide, m-chloroperbenzoic acid, meta sodium periodate, meta potassium periodate or the like in a solvent such as methylene chloride, chloroform, 1,2-dichloroethane, tetrahydrofuran, 1,4-dioxane, methanol, acetone, and water, or a mixed solvent thereof, at a temperature ranging from 0°C to the reflux temperature of a solvent, after protecting at need the nitrogen atom of the amino group represented by $R^3$ which may be substituted or the saturated nitrogen-containing heterocyclic group represented by $R^3$ whose nitrogen atom not bound to the adjacent $(CH_2)_k$ group which may be substituted, with a protecting group such as alkanoyl groups in a conventional manner, and furthermore deprotecting at need the protecting group such as alkanoyl groups in a conventional manner.

Then, the compound of the general formula (I) wherein $R^2$ is cyano group can be obtained by treating the compound of the general formula (XVII) with cyanotrimethylsilane in the presence of 1,8-diazabicyclo[5.4.0]-7-undecene in a solvent such as N,N-dimethylformamide, tetrahydrofuran, 1,4-dioxane, 1,2-dichloroethane, acetonitrile, and toluene at a temperature ranging from 0°C to the reflux temperature of a solvent.

According to the third synthetic method of the compounds of the present invention, the compound of the general formula (I) wherein $R^2$ is mercapto group can be obtained by treating the compound of the following general formula (XVIII) obtainable from a starting material wherein $R^7$ of the compound of the aforementioned general formula (V) is replaced with a chlorine atom in a similar manner to the first synthetic method:

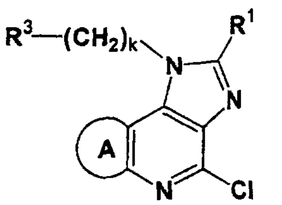

(XVIII)

wherein $R^1$, $R^3$, k, and ring A have the same meanings as those defined above, with thiourea in a solvent such as methanol, ethanol, n-propanol, N,N-dimethylformamide, dimethyl sulfoxide, tetrahydrofuran, 1,4-dioxane, or the aforementioned solvent containing water at a temperature ranging from room temperature to the reflux temperature of a solvent.

According to the forth synthetic method of the compounds of the present invention, the compound of the aforementioned general formula (I) wherein $R^2$ is carbamoyl group or carboxyl group can be obtained by treating the compound of the aforementioned general formula (I) wherein $R^2$ is cyano group obtained by the second synthetic method by using an acid such as hydrochloric acid, hydrobromic acid, sulfuric acid, and phosphoric acid or a base such as sodium hydroxide, potassium hydroxide, barium hydroxide in a solvent such as methanol, ethanol, n-propanol, ethyleneglycol, diethyleneglycol, N,N-dimethylformamide, dimethyl sulfoxide, acetic acid, water, or a mixed solvent thereof at a temperature ranging from room temperature to the reflux temperature of a solvent.

According to the fifth synthetic method of the compounds of the present invention, the compound of the aforementioned general formula(I), wherein $R^3$ is a deprotected amino group or $R^3$ is a saturated nitrogen-containing heterocyclic group whose nitrogen atom not bound to the adjacent $(CH_2)_k$ group is deprotected, can be obtained by subjecting the compound of the aforementioned general formula (I), wherein the nitrogen atom of the amino group represented by $R^3$ has a protective group such as alkanoyl group, alkoxycarbonyl group, benzyl group, and trifluoroacetyl group or wherein a nitrogen atom of the saturated nitrogen-containing heterocyclic group that is not bound to the adjacent $(CH_2)_k$ group has a protective group such as alkanoyl group, alkoxycarbonyl group, benzyl group, and trifluoroacetyl group, to deprotection reaction by using an acid or an alkali, or to hydrogenation by using a metal catalyst depending on a kind of a protective group on the nitrogen atom.

The deprotection by using an acid or an alkali can be carried out with an acid or a base in the presence or absence of a cation scavenger such as anisole and thioanisole in a solvent. Examples of the solvent used include, for example, ethyl acetate, methylene chloride, 1,2-dichloroethane, 1,4-dioxane, methanol, ethanol, n-propanol, N,N-dimethylformamide, tetrahydrofuran, water, and a mixed solvent thereof. Examples of the acid used include, for example, hydrochloric acid, an ethyl acetate solution of hydrogen chloride, an ethanolic solution of hydrogen chloride, sulfuric acid, hydrobromic acid, trifluoroacetic acid, p-toluenesulfonic acid, formic acid, acetic acid and the like. Examples of the base include, for example, hydroxides, carbonates or hydrogencarbonates of an alkali metal such as sodium and potassium or of alkaline-earth metal such as magnesium and calcium and the like. The reaction can be carried out at a temperature ranging from 0°C to the reflux temperature of a solvent.

The hydrogenation can be carried out by using a metal catalyst such as platinum, palladium/carbon, Raney nickel, Pearlman's reagent in a solvent such as water, methanol, ethanol, n-propanol, acetic acid, and a mixed solvent thereof in the presence or absence of an acid such as hydrochloric acid at a temperature ranging from room temperature to the reflux temperature of the solvent under a hydrogen pressure ranging from normal pressure to 200 Pa.

According to sixth synthetic method of the compounds of the present invention, the compound of the aforementioned general formula(I), wherein $R^3$ is an amino group which is substituted by a functional group or $R^3$ is a saturated nitrogen-containing heterocyclic group whose nitrogen atom not bound to the adjacent $(CH_2)_k$ group is substituted by a functional group, can be obtained by reacting the compound of the aforementioned general formula (I), wherein $R^3$ is non-protected amino group or wherein $R^3$ is a saturated nitrogen-containing heterocyclic group whose nitrogen atom not bound to the adjacent $(CH_2)_k$ group is not protected, with a reagent for introducing a functional group on a nitrogen atom.

The reaction can be carried out in the presence or absence of a solvent such as N,N-dimethylformamide, methylene chloride, tetrahydrofuran, toluene, pyridine, nitrobenzene, 1,2-dichloroethane, 1,4-dioxane, methanol, ethanol, n-propanol, water, and a mixed solvent thereof in the presence or absence of a base such as triethylamine and potassium carbonate at a temperature ranging from 0°C to 200°C.

Examples of the reagent for introducing a functional group on a nitrogen atom include, for example, alkyl halides, triphenylmethyl chloride, triphenylmethyl bromide, benzyl chloride, benzyl bromide, benzhydryl chloride, benzhydryl bromide, a mixture of formic acid and formalin, acetyl chloride, acetic anhydride, trifluoroacetic anhydride, benzoyl chloride, chloroacetyl chloride, benzyl chlorocarbonate, ethyl chlorocarbonate, di-tert-butyl dicarbonate, sodium cyanate, alkyl isocyanates, sodium thiocyanate, alkyl isothiocyanates, 1H-pyrazole-1-carboxamidine, methanesulfonyl chloride, p-toluenesulfonyl chloride, p-fluorobenzenesulfonyl chloride, urethanes, alkylurethanes, thiourethanes, alkylthiourethanes and the like.

According to the seventh synthetic method of the compounds of the present invention, the compound of the aforementioned general formula(I), wherein $R^3$ is an amino group having an alkoxycarbonyl group or benzyloxycarbonyl group as a substituent or $R^3$ is a saturated nitrogen-containing heterocyclic group whose nitrogen atom not bound to the adjacent $(CH_2)_k$ group has an alkoxycarbonyl group or benzyloxycarbonyl group as a substituent, can be obtained by reacting the compound of the aforementioned general formula (I), wherein $R^3$ is an amino group having an alkyl group or benzyl group as a substituent or wherein $R^3$ is a saturated nitrogen-containing heterocyclic group whose nitrogen atom not bound to the adjacent $(CH_2)_k$ group has an alkyl group or benzyl group as a substituent, with an alkyl chlorocarbonate or benzyl chlorocarbonate in the presence or absence of a solvent such as methylene chloride and toluene in the presence or absence of a base such as triethylamine and potassium carbonate at a temperature ranging from 0°C to 200°C.

According to the eighth synthetic method of the compounds of the present invention, the compound of the aforementioned general formula(I), wherein $R^3$ is an amino group having an aminoalkyl group or an aminoalkanoyl group as a substituent or wherein $R^3$ is a saturated nitrogen-containing heterocyclic group whose nitrogen atom not bound to the adjacent $(CH_2)_k$ group has an aminoalkyl group or an aminoalkanoyl group as a substituent, can be obtained by reacting the compound of the aforementioned general formula (I), wherein $R^3$ is an amino group having a halogenoalkyl group or a halogenoalkanoyl group as a substituent or wherein $R^3$ is a saturated nitrogen-containing heterocyclic group whose nitrogen atom not bound to the adjacent $(CH_2)_k$ group has a halogenoalkyl group or a halogenoalkanoyl group as a substituent, with a various kind of amines such as dimethylamine, methylamine, benzylamine and the like in the presence or absence of a solvent such as methanol, ethanol, N,N-dimethylformamide, methylene chloride, and toluene at a temperature ranging from 0°C to 200°C. Alternatively, the aforementioned compound can be obtained by treatment with potassium phthalimide in the presence or absence of a solvent such as N,N-dimethylformamide and dimethyl sulfoxide at a temperature ranging from 0°C to 200°C, and then treatment with hydrazine hydrate in the presence or absence of a solvent such as methanol, ethanol, and N,N-dimethylformamide at a temperature ranging from 0°C to 200°C.

In the preparations of the compounds of the present invention, 4-hydroxy-3-nitropyridine derivatives represented by the aforementioned general formula (V), which are used as starting materials, can be obtained, for example, by the novel synthetic method as described below:

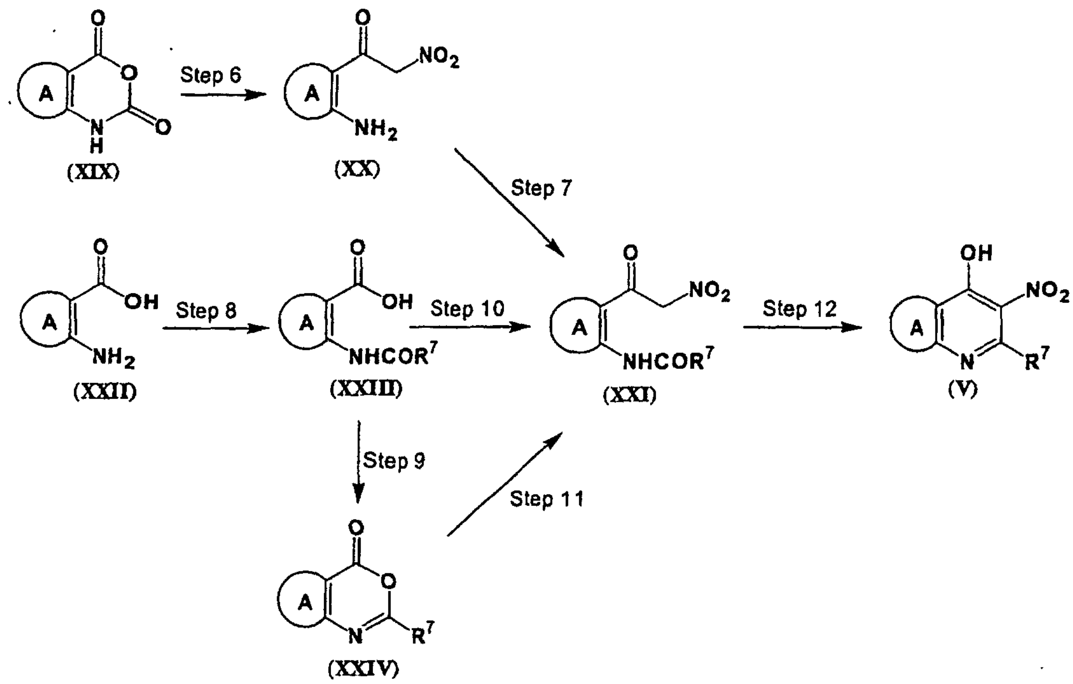


(XIX) (XX) (XXII) (XXIII) (XXI) (V) (XXIV)

wherein $R^7$ and ring A have the same meanings as those defined above.

In Step 6, the compound of the general formula (XX) can be obtained by reacting the compound of the general formula (XIX) with nitromethane in a solvent such as N,N-dimethylformamide, dimethyl sulfoxide, tetrahydrofuran, and toluene in the presence of a base such as sodium carbonate, potassium carbonate, potassium tert-butoxide, sodium hydride at a temperature ranging from 0°C to the reflux temperature of a solvent.

In Step 7, the compound of the general formula (XXI) can be obtained by reacting the compound of the general formula (XX) with the compound of the following general formula (XXV) or (XXVI):

$$R^7COX \qquad (XXV)$$

$$(R^7CO)_2O \qquad (XXVI)$$

wherein $R^7$ and X have the same meanings as those defined above, in the presence or absence of a base such as triethylamine and potassium carbonate in the presence or absence of a solvent such as methylene chloride, 1,2-dichloroethane, N,N-dimethylformamide, tetrahydrofuran, acetonitrile, xylene, and toluene at a temperature ranging from 0°C to 200°C.

As an alternative step for Step 7, the compound of the general formula (XXI) can be obtained by reacting the compound of the following general formula (XXVII):

$$R^7CO_2H \qquad (XXVII)$$

wherein $R^7$ has the same meanings as that defined above, with a reagent for activating carboxylic acid in a conventional manner to obtain an acid halide, or a mixed acid anhydride or the like, and then reacting the product with the compound of the general formula (XX) in the presence or absence of a base such as triethylamine or potassium carbonate in a solvent such as methylene chloride, 1,2-dichloroethane, N,N-dimethylformamide, tetrahydrofuran, acetonitrile, xylene, and toluene at a temperature ranging from 0°C to the reflux temperature of a solvent.

Examples of the reagent for activating carboxylic acid used in the aforementioned preparation method include, for example, thionyl chloride, oxalyl chloride, ethyl chloroformate, pivaloyl chloride, 1,1'-carbonyldiimidazole, 1,3-dicyclohexylcarbodiimide, propylphosphonic acid anhydride and the like.

As still further alternative method for preparation of the compound of the general formula (XXI), the compound of the general formula (XXII) in Step 8 is reacted with a compound with an activated carboxylic acid derived from the compound of the general formula (XXV), (XXVI), or (XXVII) in the presence or absence of a solvent such as chloroform, 1,2-dichloroethane, N,N-dimethylformamide, tetrahydrofuran, acetonitrile, xylene, and toluene at a temperature ranging from 0°C to 200°C to obtain the compound of the general formula (XXIII), and after the resulting product is activated as a pretreatment for Step 10 by using a reagent for activating carboxylic acid according to the method of Step 7, or the resulting product is dehydrated for Step 9 by a method of heating in acetic anhydride and the like to obtain the compound of the general formula (XXIV), the compound of the general formula (XXI) can be obtained by reacting the compound of general formula (XXIII) or (XXIV) with nitromethane for Step 10 or Step 11 in a solvent such as N,N-dimethylformamide, dimethyl sulfoxide, tetrahydrofuran, acetonitrile, and toluene in the presence of a base such as sodium carbonate, potassium carbonate, potassium tert-butoxide, sodium hydride at a temperature ranging from 0°C to the reflux temperature of a solvent.

In Step 12, the compound of the general formula (V) can be obtained by treating the compound of the general formula (XXI) in the presence of a base such as 4-dimethylaminopyridine, sodium carbonate, potassium carbonate, potassium tert-butoxide, and sodium hydride in a solvent such as N,N-dimethylformamide, tetrahydrofuran, and acetonitrile at a temperature ranging from 0°C to the reflux temperature of a solvent.

Some of the compounds represented by the general formulas (VII), (XVI), and (XVIII) to (XXIV) which are starting materials or synthetic intermediates in the preparations of the compounds of the present invention are known compounds, which are disclosed in, for example, Journal of Medicinal Chemistry, Vol. 40, p.1779 (1997); Chemical Pharmaceutical Bulletin, Vol. 24, p.431, 1976; Synthesis, p.505, 1980; The Journal of Organic Chemistry, Vol. 50, p. 1246, 1985; Journal of Heterocyclic Chemistry, Vol. 21, p. 1345, 1984 and the like, and can be prepared according to the methods described therein. The preparations of some novel compounds will be described in reference examples.

The compounds of the present invention have inhibitory action against production of a cytokine, and they are useful as active ingredients of medicaments for preventive and/or therapeutic treatment of diseases in which a cytokine such as TNF or IL-1 is involved. Examples of the diseases in which a cytokine is involved include, for example, chronic inflammatory diseases (e.g., rheumatic arthritis, osteoarthritis and the like), allergic rhinitis, atopic dermatitis, contact dermatitis, urticaria, eczema, pruritus cutaneus, prurigo, asthma, sepsis, septic shock, various autoimmune diseases [autoimmune hemic diseases (e.g., hemolytic anemia, anaplastic anemia, idiopathic thrombocythemia and the like), autoimmune intestinal diseases (e.g., ulcerative colitis, Crohn's disease and the like), autoimmune corneitis (e.g., keratoconjunctivitis sicca, spring catarrh and the like), endocrine ophthalmopathy, Graves disease, sarcoid granuloma, multiple sclerosis, systemic erythematodes, multiple chondritis, pachydermia, active chronic hepatitis, myasthenia gravis, psoriasis, interstitial pulmonary fibrosis and the like], diabetes, cancerous cachexia, HIV-infectious cachexia and the like of mammals including human.

The medicaments which comprise as an active ingredient the novel 1H-imidazopyridine derivative represented by the aforementioned general formula (I) or (II) or a pharmacologically acceptable salt thereof are generally administered as oral preparations in the forms of capsules, tablets, fine granules, granules, powders, syrups, dry syrups, solutions and the like, or as parenteral preparations in the forms of injections, suppositories, eye drops, eye ointments, ear drops, nasal drops, dermal preparations, inhalations and the like. These formulations can be manufactured according to conventional methods by addition of pharmacologically and pharmaceutically acceptable additives. For example, in the oral preparations and suppositories, pharmaceutical ingredients may be used such as excipients such as lactose, D-mannitol, corn starch, and crystalline cellulose; disintegrators such as carboxymethylcellulose, carboxymethylcellulose calcium, partly pregelatinized starch, croscarmellose sodium, and crospovidone; binders such as hydroxypropyl cellulose, hydroxypropyl methylcellulose, and polyvinylpyrrolidone; lubricants such as magnesium stearate, talc, hydrogenated oil, dimethylpolysiloxane, hydrated silicon dioxide, light anhydrous silicic acid, and carnauba wax; coating agents such as hydroxypropyl methylcellulose, sucrose, and titanium oxide; plasticizers such as triethyl citrate, polyethylene glycol, and fatty acid ester of glycerol; bases such as polyethylene glycol and hard fat and the like. In injections, or eye or ear drops, pharmaceutical ingredients may be used such as solubilizers or solubilizing aids which may constitute aqueous preparations or those dissolved upon use such as distilled water for injection, physiological saline, and propylene glycol; pH modifiers such as inorganic or organic acids or bases; isotonicities such as sodium chloride, glucose, and glycerin; stabilizers and the like; and in eye ointments and dermal preparations, pharmaceutical ingredients which are suitable for ointments, creams and patches such as white vaseline, macrogols, glycerin, and cotton cloth.

A dose of the medicament of the present invention may be appropriately chosen depending on conditions of a patient and a route of administration. For example, generally from about 0.1 to 1,000 mg for oral administration, and from about 0.01 to 500 mg for parenteral administration as a daily dose for an adult, which may be administered one

a day or several times a day as divided portions. However, it is desirable that the aforementioned dose may suitably be increased or decreased depending on a purpose of a therapeutic or preventive treatment, part or type of a disease, and the age or symptoms of a patient.

Examples

The present invention will be more specifically explained by referring to Reference Examples and Working Examples. However, the scope of the present invention is not limited to these examples.

The abbreviations in the tables have the following meanings: Ph, phenyl group; Boc, tert-butoxycarbonyl group; Me, methyl group; and Et, ethyl group.

Reference example 1

2'-Amino-4'-chloro-2-nitroacetophenone

To a solution of 14.4 g of 7-chloro-2H-3,1-benzoxazine-2,4-1H-dione in 120 ml of dimethyl sulfoxide, 20.1 g of potassium carbonate and 15.7 ml of nitromethane were added, and the mixture was stirred at 40°C for 24 hours. The reaction mixture was added with 8 ml of nitromethane again, and stirred at 40°C for 24 hours. The reaction mixture was poured into ice-water and adjusted to pH 5 with 10% hydrochloric acid, and then added with diethyl ether. The insoluble matter was filtered off, and the diethyl ether layer was washed with water, and dried, and the solvent was evaporated. The residue was purified by silica gel column chromatography using a mixture of ethyl acetate and n-heptane (1:1) as eluting solvent, and then washed with diisopropyl ether to give 7.01 g of yellowish brown crystals. Recrystallization from a mixture of ethyl acetate and diisopropyl ether gave yellowish orange crystals having the melting point of from 131 to 132°C.
Elemental analysis for $C_8H_7ClN_2O_3$
Calculated % C,44.77; H,3.29; N,13.05
Found % C,44.73; H,3.27; N,12.99

In accordance with the method of Reference example 1, the compounds of Reference examples 2 through 4 were obtained.

Reference example 2

2'-Amino-4'-fluoro-2-nitroacetophenone

Appearance : pale yellowish brown needles
Recrystallization solvent : ethyl acetate-diisopropyl ether
Melting point: 117-118°C
Elemental analysis for $C_8H_7FN_2O_3$
Calculated % C,48.49; H,3.56; N,14.14
Found % C,48.71; H,3.68; N,14.20

Reference example 3

2'-Amino-4'-methyl-2-nitroacetophenone

Appearance : yellow crystals
Recrystallization solvent : ethyl acetate-diisopropyl ether
Melting point : 96-97°C
Elemental analysis for $C_9H_{10}N_2O_3$
Calculated % C,55.67; H,5.19; N,14.43
Found %C,55.69; H,5.04; N,14.42

Reference example 4

2'-Amino-3'-methyl-2-nitroacetophenone

Appearance : yellow crystals
Recrystallization solvent: ethyl acetate-diisopropyl ether
Melting point: 100-101°C
Elemental analysis for $C_9H_{10}N_2O_3$
Calculated % C,55.67; H,5.19; N,14.43
Found % C,55.67; H,5.07; N,14.43

Reference example 5

2-Nitro-2'-(trifluoroacetylamio)acetophenone

To a suspension of 7.65 g of 2'-amino-2-nitroacetophenone in 50 ml of toluene, 6 ml of trifluoroacetic anhydride was added dropwise under ice-cooling, and the mixture was stirred at room temperature for 30 minutes. The reaction mixture was added with water, and extracted with ethyl acetate. The extract was washed successively with water and saturated brine, and dried, and the solvent was evaporated. The obtained residue was washed with diisopropyl ether to give 10.7 g of pale purple crystals.
NMR spectrum δ ($CDCl_3$) ppm: 5.96 (2H,s), 7.36 (1H,t,J=8Hz) 7.68 (1H,d,J=8Hz), 7.79 (1H,t,J=8Hz), 8.80 (1H,d, J=8Hz), 12.14 (1H,brs)
IR spectrum ν (KBr)$cm^{-1}$:1734,1680
Mass spectrum m/z:276($M^+$)

Reference example 6

2'-(Benzoylamino)-2-nitroacetophenone

The mixture of 5.72 g of 2-phenyl-4H-3,1-benzoxazin-4-one, 4.96 g of potassium carbonate, 2.1 ml of nitromethane and 29 ml of dimethyl sulfoxide was stirred at room temperature for 3 hours. The reaction liquid was poured into water and added with ethyl acetate, and then adjusted to pH 3-4 with 2 M hydrochloric acid dropwise. The precipitated crystals were collected by filtration. The crystals were washed successively with water and ethyl acetate to give 5.94 g of pale yellow crystals.
NMR spectrum δ ($CDCl_3$) ppm: 6.00 (2H,s), 7.21 (1H,t,J=7Hz), 7.54 (2H,t,J=7Hz), 7.59(2H,t,J=8Hz), 7.75 (1H,t,J=8Hz), 8.05 (2H,d,J=7Hz), 9.09 (1H,d,J=8Hz), 12.08(1H,brs)
IR spectrum ν (KBr)$cm^{-1}$:3312,1692,1668,1590,1306
Mass spectrum m/z:284($M^+$)
In accordance with the methods of Reference examples 5 and 6, the compounds of Reference examples 7 through 14 were obtained.

| Reference example | Structural formula | Physical properties (Recrystallization solvent) |
|---|---|---|
| 7 | 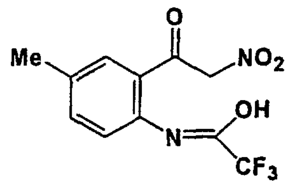 | yellowish orange crystals<br>NMR δ ($CDCl_3$)ppm:2.32(3H,s),5.48(1H, brs),6.81(1H,d,J=8Hz),7.35(1H,dd,J=8,2 Hz),7.73(1H,d,J=2Hz)<br>IR ν (KBr) $cm^{-1}$:3352,1738<br>MS m/z:289($M^+$-1) |
| 8 | 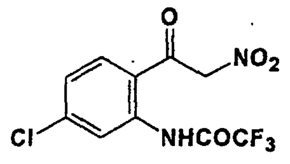 | pale yellow crystals<br>(AcOEt-iso-$Pr_2O$)<br>mp, 152-153°C<br>Elemental analysis for $C_{10}H_6ClF_3N_2O_4$<br>Calcd.%: C, 38.67; H, 1.95; N, 9.02<br>Found%: C, 38.84; H, 1.97; N, 8.98 |
| 9 | 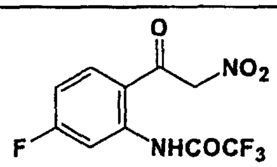 | pale yellow needles(iso-$Pr_2O$)<br>mp, 129-130°C<br>Elemental analysis for $C_{10}H_6F_4N_2O_4$<br>Calcd.%: C, 40.83; H, 2.06; N, 9.52<br>Found%: C, 40.79; H, 2.10; N, 9.58 |
| 10 | 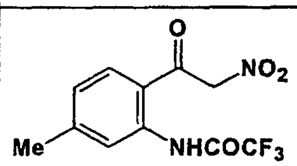 | colorless crystals(AcOEt-iso-$Pr_2O$)<br>mp, 161-162°C<br>Elemental analysis for $C_{11}H_9F_3N_2O_4$<br>Calcd.%: C, 45.53; H, 3.13; N, 9.65<br>Found%: C, 45.53; H, 3.05; N, 9.77 |
| 11 | 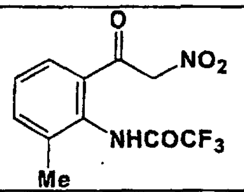 | colorless crystals(AcOEt-iso-$Pr_2O$)<br>mp, 131.5-133°C<br>Elemental analysis for $C_{11}H_9F_3N_2O_4$<br>Calcd.%: C, 45.53; H, 3.13; N, 9.65<br>Found%: C, 45.55; H, 3.07; N, 9.61 |
| 12 | 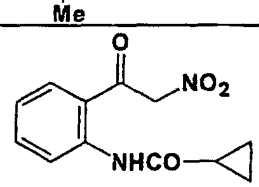 | colorless crystals(AcOEt-iso-$Pr_2O$)<br>mp, 139.5-140.5°C<br>Elemental analysis for $C_{12}H_{12}N_2O_4$<br>Calcd.%: C, 58.06; H, 4.87; N, 11.29<br>Found%: C, 57.99; H, 4.89; N, 11.32 |
| 13 | 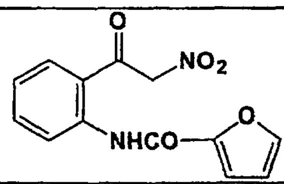 | colorless fine needles(MeOH)<br>mp, 196.5-198.5°C (decomposition)<br>Elemental analysis for $C_{13}H_{10}N_2O_5$<br>Calcd.%: C, 56.94; H, 3.68; N, 10.22<br>Found%: C, 56.95; H, 3.76; N, 10.25 |
| 14 | 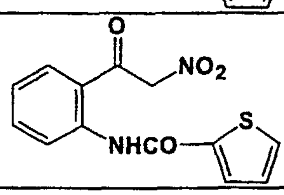 | pale green crystals(MeOH)<br>mp, 176.5-177°C (decomposition)<br>Elemental analysis for $C_{13}H_{10}N_2O_4S$<br>Calcd.%: C, 53.79; H, 3.47; N, 9.65<br>Found%: C, 53.71; H, 3.55; N, 9.61 |

Reference example 15

3-Nitro-2-trifluoromethyl-4-quinolinol

A solution of 10.5 g of 2-nitro-2'-(trifluoroacetylamino)acetophenone and 5.57 g of 4-dimethylaminopyridine in 80 ml of tetrahydrofuran was refluxed for 30 minutes. The reaction mixture was added with water and adjusted to

pH 1 with 10% hydrochloric acid. The precipitated crystals were collected by filtration and washed successively with water and diisopropyl ether to give 9.50 g of crystals. Recrystallization from ethyl acetate gave pale brown crystals having the sublimation point of from 245 to 254°C.
Elemental analysis for $C_{10}H_5F_3N_2O_3$
Calculated % C,46.53; H,1.95; N,10.85
Found % C,46.40; H,2.12; N,10.95

In accordance with the method of Reference example 15, the compounds of Reference examples 16 through 24 were obtained.

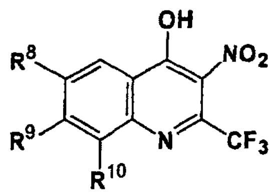

| Reference example | R 8 | R 9 | R10 | Physical properties (Recrystallization solvent) |
|---|---|---|---|---|
| 16 | Me | H | H | yellowish brown crystals(EtOH-AcOEt) mp, 264-266.5°C (decomposition)<br>Elemental analysis for $C_{11}H_7F_3N_2O_3$<br>Calcd.%: C, 48.54; H, 2.59; N, 10.29<br>Found%: C, 48.53; H, 2.78; N, 10.39 |
| 17 | H | Cl | H | colorless crystals(AcOEt) mp, 237-249°C (sublimation)<br>Elemental analysis for $C_{10}H_4ClF_3N_2O_3$<br>Calcd.%: C, 41.05; H, 1.38; N, 9.57<br>Found%: C, 40.94; H, 1.46; N, 9.52 |
| 18 | H | F | H | pale orange plates(AcOEt-iso-$Pr_2O$) mp, 270-273°C (decomposition)<br>Elemental analysis for $C_{10}H_4F_4N_2O_3$<br>Calcd.%: C, 43.49; H, 1.46; N, 10.14<br>Found%: C, 43.61; H, 1.67; N, 10.28 |
| 19 | H | Me | H | pale yellowish brown crystals(AcOEt) mp, 222-224°C (sublimation)<br>Elemental analysis for $C_{11}H_7F_3N_2O_3$<br>Calcd.%: C, 48.54; H, 2.59; N, 10.29<br>Found%: C, 48.58; H, 2.61; N, 10.35 |
| 20 | H | H | Me | yellow crystals(n-Heptane-iso-$Pr_2O$) mp, 156-157°C<br>Elemental analysis for $C_{11}H_7F_3N_2O_3$<br>Calcd.%: C, 48.54; H, 2.59; N, 10.29<br>Found%: C, 48.82; H, 2.76; N, 10.37 |

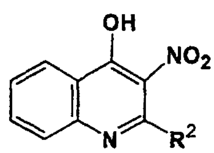

| Reference example | $-R^2$ | Physical properties (Recrystallization solvent) |
|---|---|---|
| 21 | 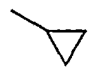 | yellowish brown crystals(MeOH)<br>mp, ≧300℃<br>Elemental analysis for $C_{12}H_{10}N_2O_3$<br>Calcd.%: C, 62.60; H, 4.38; N, 12.17<br>Found%: C, 62.48; H, 4.47; N, 12.14 |
| 22 | -Ph | pale yellow crystals(MeOH)<br>mp, ≧300℃<br>Elemental analysis for $C_{15}H_{10}N_2O_3$<br>Calcd.%: C, 67.67; H, 3.79; N, 10.52<br>Found%: C, 67.44; H, 3.88; N, 10.44 |
| 23 | 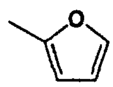 | yellow crystals(MeOH)<br>mp, ≧300℃<br>Elemental analysis for $C_{13}H_8N_2O_4$<br>Calcd.%: C, 60.94; H, 3.15; N, 10.93<br>Found%: C, 61.00; H, 3.29; N, 10.91 |
| 24 | 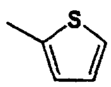 | yellow prisms(MeOH)<br>mp, ≧300℃<br>Elemental analysis for $C_{13}H_8N_2O_3S$<br>Calcd.%: C, 57.35; H, 2.96; N, 10.29<br>Found%: C, 57.27; H, 3.13; N, 10.23 |

Reference example 25

4-Chloro-3-nitro-2-trifluoromethylquinoline

A mixture of 12.6 g of 3-nitro-2-trifluoromethyl-4-quinolinol and 50 ml of phosphorus oxychloride was stirred at 100°C for 2 hours. The reaction mixture was left to cool, and poured into ice, and the precipitated crystals were collected by filtration. The collected crystals were dissolved in toluene, and washed successively with water and saturated brine, and dried, and the solvent was evaporated. The residue was washed with n-heptane to give 12.6 g of pale purple crystals. Recrystallization from n-heptane gave colorless crystals having the melting point of from 119 to 120°C.
Elemental analysis for $C_{10}H_4ClF_3N_2O_2$
Calculated % C,43.42; H,1.46; N,10.13
Found % C,43.32; H,1.63; N,10.16
In accordance with the method of Reference example 25, the compounds of Reference examples 26 through 34 were obtained.

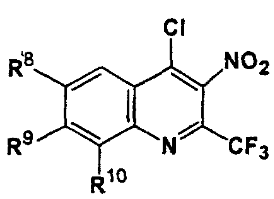

| Reference example | $R^8$ | $R^9$ | $R^{10}$ | Physical properties (Recrystallization solvent) |
|---|---|---|---|---|
| 26 | Me | H | H | pale yellow crystals(n-Heptane) mp, 108.5-109.5°C<br>Elemental analysis for $C_{11}H_6ClF_3N_2O_2$<br>Calcd.%: C, 45.46; H, 2.08; N, 9.64<br>Found%: C, 45.27; H, 2.22; N, 9.52 |
| 27 | H | Cl | H | pale yellow needles(n-Heptane) mp, 91-91.5°C<br>Elemental analysis for $C_{10}H_3Cl_2F_3N_2O_2$<br>Calcd.%: C, 38.61; H, 0.97; N, 9.01<br>Found%: C, 38.85; H, 1.19; N, 9.07 |
| 28 | H | F | H | pale yellow needles(n-Heptane) mp, 52-53°C<br>Elemental analysis for $C_{10}H_3ClF_4N_2O_2$<br>Calcd.%: C, 40.77; H, 1.03; N, 9.51<br>Found%: C, 40.60; H, 1.28; N, 9.55 |
| 29 | H | Me | H | colorless crystals(n-Heptane) mp, 110.5-111.5°C<br>Elemental analysis for $C_{11}H_6ClF_3N_2O_2$<br>Calcd.%: C, 45.46; H, 2.08; N, 9.64<br>Found%: C, 45.32; H, 2.22; N, 9.60 |
| 30 | H | H | Me | colorless needles(n-Heptane) mp, 128-129°<br>Elemental analysis for $C_{11}H_6ClF_3N_2O_2$<br>Calcd.%: C, 45.46; H, 2.08; N, 9.64<br>Found%: C, 45.34; H, 2.28; N, 9.63 |

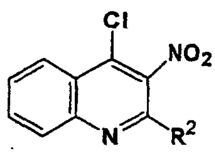

| Reference example | $-R^2$ | Physical properties (Recrystallization solvent) |
|---|---|---|
| 31 | 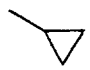 | colorless crystals(n-Heptane)<br>mp,116-117°C<br>Elemental analysis for $C_{12}H_9ClN_2O_2$<br>Calcd.%: C, 57.96; H, 3.65; N, 11.27<br>Found%: C, 57.94; H, 3.76; N, 11.28 |
| 32 | -Ph | colorless prisms (AcOEt-n-Heptane)<br>mp,149.5-151°C<br>Elemental analysis for $C_{15}H_9ClN_2O_2$<br>Calcd.%: C, 63.28; H, 3.19; N, 9.84<br>Found%: C, 63.03; H, 3.38; N, 9.81 |
| 33 | 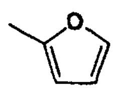 | pale brown crystals(iso-$Pr_2O$)<br>mp,143.5-144.5°C<br>Elemental analysis for $C_{13}H_7ClN_2O_3$<br>Calcd.%: C, 56.85; H, 2.57; N, 10.20<br>Found%: C, 56.69; H, 2.76; N, 10.11 |
| 34 | 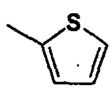 | brown crystals(iso-$Pr_2O$)<br>mp,105-107°C<br>Elemental analysis for $C_{13}H_7ClN_2O_2S$<br>Calcd.%: C, 53.71; H, 2.43; N, 9.64<br>Found%: C, 53.76; H, 2.61; N, 9.58 |

Reference example 35

tert-Butyl exo-3-ethoxycarbonyl-8-azabicyclo[3.2.1]octane-8-carboxylate

To a solution of 10.3 g of ethyl exo-8-azabicyclo[3.2.1]octane-3-carboxylate in 30 ml of methanol, under stirring and ice-cooling, a solution of 13.5 g of di-tert-butyl dicarbonate in 40 ml of methanol was added dropwise, and the mixture was stirred at room temperature for 1.5 hours. After the reaction, the solvent was evaporated, and the residue was dissolved in diethyl ether and washed with saturated brine, and dried, and the solvent was evaporated to give 16.0 g of a pale yellow liquid.
NMR spectrum δ ($CDCl_3$) ppm:1.24 (3H,t,J=7.5Hz), 1.47 (9H,s), 1.58-1.77 (4H,m), 1.79-2.06 (4H,m), 2.75-2.84 (1H,m), 4.11 (2H,q,J=7.5Hz), 4.13-4.37(2H,m)
IR spectrum ν (liq.)$cm^{-1}$:1736,1698

Reference example 36

tert-Butyl exo-3-hydroxymethyl-8-azabicyclo[3.2.1]octane-8-carboxylate

To a solution of 15.8 g of tert-butyl exo-3-ethoxycarbonyl-8-azabicyclo[3.2.1]octane-8-carboxylate in 65 ml of tetrahydrofuran, 6.30 g of sodium borohydride was added, and then a mixture of 40 ml of methanol and 40 ml of tetrahydrofuran was added dropwise under stirring at room temperature. The mixture was stirred overnight at room temperature, and the solvent was evaporated. The residue was added with water, and extracted with toluene. The extract was washed with saturated brine, and dried, and the solvent was evaporated to give 13.3 g of a colorless viscous liquid.
NMR spectrum δ ($CDCl_3$) ppm:1.29-1.78 (7H,m),1.46(9H,s), 1.88-2.13 (3H,m), 3.44 (2H,brs), 4.12-4.35 (2H,m)
IR spectrum ν (liq.)$cm^{-1}$: 1696

Reference example 37

tert-Butyl exo-3-(methanesulfonyloxymethyl)-8-azabicyclo[3.2.1]octane-8-carboxylate

To a solution of 14.6 g of tert-butyl exo-3-hydroxymethyl-8-azabicyclo[3.2.1]octane-8-carboxylate in 60 ml of tetrahydrofuran, under stirring and ice-cooling, 10.0 ml of triethylamine was added, and then 4.9 ml of methanesulfonyl chloride in 10 ml of tetrahydrofuran was added dropwise. The mixture was stirred under ice-cooling for 20 minutes. The reaction mixture was added with ice-water and extracted with toluene. The extract was washed with saturated brine, and dried, and the solvent was evaporated to give 19.3 g of a pale yellow liquid.
NMR spectrum δ ($CDCl_3$) ppm:1.36-1.71 (6H,m), 1.47 (9H,s), 1.92-2.06 (2H,m), 2.25-2.37 (1H,m), 2.99 (3H,s), 4.00 (2H,d,J=6.5Hz), 4.15-4.35 (2H,m)
IR spectrum ν (liq.)$cm^{-1}$: 1692

Reference example 38

tert-Butyl exo-3-cyanomethyl-8-azabicyclo[3.2.1]octane-8-carboxylate

To a solution of 19.1 g of tert-butyl exo-3-(methanesulfonyloxymethyl)-8-azabicyclo[3.2.1]octane-8-carboxylate in 90 ml of dimethyl sulfoxide, 6.30 g of sodium cyanide and 0.90 g of sodium iodide were added successively, and the mixture was stirred at 90°C for 2 hours. The reaction mixture was added with ice-water and extracted with toluene. The extract was washed with saturated brine, and dried, and the solvent was evaporated to give 14.3 g of a pale yellowish brown liquid.
NMR spectrum δ ($CDCl_3$) ppm:1.39-1.79 (6H,m),1.47(9H,s), 1.90-2.07 (2H,m), 2.15-2.32 (3H,m), 4.13-4.37 (2H,m)
IR spectrum ν (liq.)$cm^{-1}$: 2248, 1694

Reference example 39

tert-Butyl exo-3-(2-aminoethyl)-8-azabicyclo[3.2.1]octane-8-carboxylate

To a solution of 14.1 g of tert-butyl exo-3-cyanomethyl-8-azabicyclo[3.2.1]octane-8-carboxylate in 400 ml of methanol, 50 ml of 20% methanol solution of ammonia and 3 ml of Raney-nickel were added, and the mixture was hydrogenated at 30°C and 50 Pa of hydrogen pressure. The catalyst was filtered off, and the solvent was evaporated to give 13.2 g of a green viscous liquid.
NMR spectrum δ ($CDCl_3$) ppm:1.25-1.75 (8H,m), 1.46 (9H,s), 1.79-2.07 (3H,m), 2.55-2.87 (2H,m), 4.03-4.40(4H,m)
IR spectrum ν (liq.)$cm^{-1}$: 1694
Mass spectrum m/z: 255($M^+$+1)

Reference example 40

tert-Butyl 4-[2-[(3-nitro-2-trifluoromethylquinolin-4-yl)amino]ethyl]-1-piperidinecarboxylate

A suspension of 3.78 g of 4-chloro-3-nitro-2-trifluoromethylquinoline, 6.24 g of tert-butyl 4-(2-aminoethyl)-1-piperidinecarboxylate and 1.89 g of potassium carbonate in 40 ml of N,N-dimethylformamide was stirred at room temperature for 1 hour. The reaction mixture was added with water and extracted with ethyl acetate. The extract was washed successively with water and saturated brine, and dried, and the solvent was evaporated. The residue was washed with diisopropyl ether to give 5.46 g of crystals. Recrystallization from diisopropyl ether gave colorless crystals having the melting point of from 135.5 to 136.5°C.
Elemental analysis for $C_{22}H_{27}F_3N_4O_4$
Calculated % C,56.40; H,5.81; N,11.96
Found % C,56.29; H,5.72; N,11.88
In accordance with the method of Reference example 40, the compounds of Reference examples 41 through 55 were obtained.

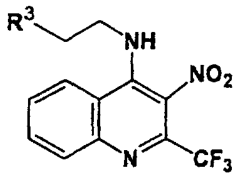

| Reference example | $R^3$- | Physical properties (Recrystallization solvent) |
|---|---|---|
| 41 | 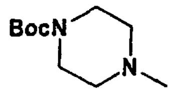 | yellow crystals(iso-$Pr_2O$)<br>mp, 148-149°C<br>Elemental analysis for $C_{21}H_{26}F_3N_5O_4$<br>Calcd.%: C, 53.73; H, 5.58; N, 14.92<br>Found%: C, 53.65; H, 5.49; N, 14.98 |
| 42 | 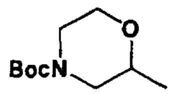 | yellow prisms(AcOEt-iso-$Pr_2O$)<br>mp, 130-131°C<br>Elemental analysis for $C_{21}H_{25}F_3N_4O_5$<br>Calcd.%: C, 53.61; H, 5.36; N, 11.91<br>Found%: C, 53.43; H, 5.20; N, 11.85 |
| 43 | 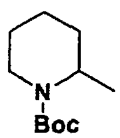 | yellow crystals(MeOH)<br>mp, 143.5-144°C<br>Elemental analysis for $C_{22}H_{27}F_3N_4O_4$<br>Calcd.%: C, 56.40; H, 5.81; N, 11.96<br>Found%: C, 56.37; H, 5.77; N, 11.93 |
| 44 | 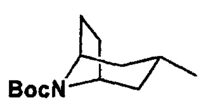 | yellow plates(AcOEt-iso-$Pr_2O$)<br>mp, 151-152°C<br>Elemental analysis for $C_{24}H_{29}F_3N_4O_4$<br>Calcd.%: C, 58.29; H, 5.91; N, 11.33<br>Found%: C, 58.23; H, 5.92; N, 11.27 |
| 45 | BocHN- | yellow needles(iso-$Pr_2O$)<br>mp ,155-156.5°C<br>Elemental analysis for $C_{17}H_{19}F_3N_4O_4$<br>Calcd.%: C, 51.00; H, 4.78; N, 13.99<br>Found%: C, 51.07; H, 4.87; N, 14.10 |
| 46 | $Me_2N$- | yellow crystals(MeOH)<br>mp, 128-129°C<br>Elemental analysis for $C_{14}H_{15}F_3N_4O_2$<br>Calcd.%: C, 51.22; H, 4.61; N, 17.07<br>Found%: C, 51.12; H, 4.63; N, 17.10 |

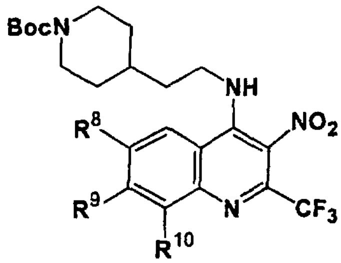

| Reference example | R 8 | R 9 | R10 | Physical properties (Recrystallization solvent) |
|---|---|---|---|---|
| 47 | Me | H | H | yellow crystals(AcOEt-iso-$Pr_2O$) mp, 168-169°C<br>Elemental analysis for $C_{23}H_{29}F_3N_4O_4$<br>Calcd.%: C, 57.25; H, 6.06; N, 11.61<br>Found%: C, 57.28; H, 5.98; N, 11.52 |
| 48 | H | Cl | H | pale yellow crystals(AcOEt-iso-$Pr_2O$) mp, 147.5-148.5°C<br>Elemental analysis for $C_{22}H_{26}ClF_3N_4O_4$<br>Calcd.%: C, 52.54; H, 5.21; N, 11.14<br>Found%: C, 52.57; H, 5.18; N, 11.12 |
| 49 | H | F | H | yellow prisms(AcOEt-iso-$Pr_2O$) mp, 159-160°C<br>Elemental analysis for $C_{22}H_{26}F_4N_4O_4$<br>Calcd.%: C, 54.32; H, 5.39; N, 11.52<br>Found%: C, 54.39; H, 5.52; N, 11.34 |
| 50 | H | Me | H | yellow crystals(AcOEt-iso-$Pr_2O$) mp, 144.5-145.5°C<br>Elemental analysis for $C_{23}H_{29}F_3N_4O_4$<br>Calcd.%: C, 57.25; H, 6.06; N, 11.61<br>Found%: C, 57.29; H, 5.99; N, 11.58 |
| 51 | H | H | Me | yellow crystals(iso-$Pr_2O$) mp, 137.5-138.5°C<br>Elemental analysis for $C_{23}H_{29}F_3N_4O_4$<br>Calcd.%: C, 57.25; H, 6.06; N, 11.61<br>Found%: C, 57.05; H, 6.00; N, 11.58 |

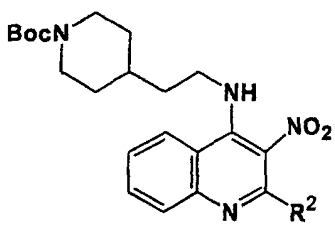

| Reference example | $-R^2$ | Physical properties (Recrystallization solvent) |
|---|---|---|
| 52 | 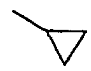 | yellow needles(MeOH)<br>mp, 149-150°C<br>Elemental analysis for $C_{24}H_{32}N_4O_4$<br>Calcd.%: C, 65.43; H, 7.32; N, 12.72<br>Found%: C, 65.35; H, 7.24; N, 12.66 |
| 53 | -Ph | yellow amorphous solid<br>NMR $\delta$ (DMSO-$d_6$)ppm:0.90-1.05(2H,m),1.37(9H,s),1.40-1.55(1H,m),1.55-1.65(4H,m),2.65(2H,t,J=12Hz),3.25(2H,q,J=5.5Hz),3.80-3.90(2H,m),7.39(1H,t,J=5.5Hz),7.40-7.50(5H,m),7.61(1H,t,J=8Hz),7.79(1H,t,J=8Hz),7.89(1H,d,J=8Hz),8.46(1H,d,J=8Hz)<br>IR $\nu$ (KBr)$cm^{-1}$:3368,1692,1530,1368 |
| 54 | 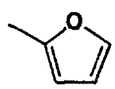 | yellow crystals(iso-$Pr_2O$)<br>mp, 125.5-127.5°C<br>Elemental analysis for $C_{25}H_{30}N_4O_5$<br>Calcd.%: C, 64.36; H, 6.48; N, 12.01<br>Found%: C, 64.30; H, 6.39; N, 11.94 |
| 55 | 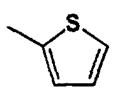 | yellow fine needles(MeOH-iso-$Pr_2O$)<br>mp, 136-137°C<br>Elemental analysis for $C_{25}H_{30}N_4O_4S$<br>Calcd.%: C, 62.22; H, 6.27; N, 11.61<br>Found%: C, 62.05; H, 6.15; N, 11.46 |

Reference example 56

tert-Butyl 4-[2-[(3-amino-2-trifluoromethylquinolin-4-yl)amino]ethyl]-1-piperidinecarboxylate

To a solution of 1.34 g of nickel chloride hexahydrate in 20 ml of methanol, under ice-cooling, 0.21 g of sodium borohydride was added portionwise and a solution of 5.30 g of tert-butyl 4-[2-[(3-nitro-2-trifluoromethylquinolin-4-yl) amino]ethyl]-1-piperidinecarboxylate in 20 ml of tetrahydrofuran and 80 ml of methanol was added. The mixture was added little by little with 1.50 g of sodium borohydride, and stirred at room temperature for 30 minutes. After the reaction, an insoluble matter was filtered off, and the solvent was evaporated. The residue was added with aqueous solution of ammonium chloride and extracted with ethyl acetate. The extract was washed with water, and dried, the solvent was evaporated to give a yellowish brown liquid. The residue was purified by silica gel column chromatography using a mixture of ethyl acetate and n-heptane (1:4 to 1:2) as eluting solvent, and washed with a mixture of diisopropyl ether and n-heptane to give 4.47 g of pale yellow crystals. Recrystallization from a mixture of diisopropyl ether and n-heptane gave colorless crystals having the melting point of from 94 to 95°C.
Elemental analysis for $C_{22}H_{29}F_3N_4O_2$
Calculated % C,60.26; H,6.67; N,12.78
Found % C,60.10; H,6.57; N,12.76

In accordance with the method of Reference example 56, the compounds of Reference examples 57 through 71 were obtained.

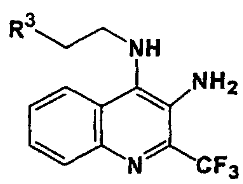

| Reference example | $R^3$- | Physical properties (Recrystallization solvent) |
|---|---|---|
| 57 | 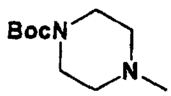 | yellow liquid<br>NMR δ ($CDCl_3$)ppm:1.48(9H,s),2.48(4H,t,J=5Hz),2.52(2H,t,J=5Hz),3.36(2H,brs),3.51(4H,t,J=5Hz),4.46(3H,brs),7.45-7.50(2H,m),7.90-7.95(1H,m),8.00-8.05(1H,m)<br>IR ν (liq.)$cm^{-1}$:3484,3364,1696<br>MS m/z:439($M^+$) |
| 58 | 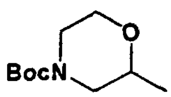 | pale yellowish brown liquid<br>NMR δ ($CDCl_3$)ppm:1.47(9H,s),1.75-1.85(2H,m),2.60-2.80(1H,m),2.90-3.10(1H,m),3.25-3.37(1H,m),3.42-3.53(1H,m),3.57-3.73(2H,m),3.77-4.07(3H,m),4.34(3H,brs),7.48-7.55(2H,m),7.83-7.88(1H,m),8.01-8.06(1H,m)<br>IR ν (liq.)$cm^{-1}$:1696<br>MS m/z:439($M^+$-1) |
| 59 | 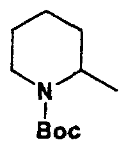 | pale yellow crystals(iso-$Pr_2O$)<br>mp, 122-123°C<br>Elemental analysis for $C_{22}H_{29}F_3N_4O_2$<br>Calcd.%: C, 60.26; H, 6.67; N, 12.78<br>Found%: C, 60.30; H, 6.55; N, 12.69 |
| 60 | 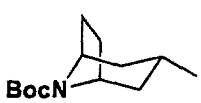 | colorless prisms(AcOEt)<br>mp, 149-150°C<br>Elemental analysis for $C_{24}H_{31}F_3N_4O_2$<br>Calcd.%: C, 62.05; H, 6.73; N, 12.06<br>Found%: C, 62.02; H, 6.77; N, 11.96 |
| 61 | BocHN- | pale green crystals(iso-$Pr_2O$-n-Heptane)<br>mp, 97.5-98°C<br>Elemental analysis for $C_{17}H_{21}F_3N_4O_2$<br>Calcd.%: C, 55.13; H, 5.72; N, 15.13<br>Found%: C, 55.09; H, 5.72; N, 15.09 |
| 62 | $Me_2N$- | yellow liquid<br>NMR δ ($CDCl_3$)ppm:2.32(6H,s),2.39(2H,t,J=5.5Hz),3.35(2H,q,J=5.5Hz),4.38(1H,brs),4.66(2H,brs),7.45-7.55(2H,m),7.95-8.00(1H,m),8.00-8.05(1H,m)<br>IR ν (liq.)$cm^{-1}$:3488,3356<br>MS m/z:299($M^+$+1) |

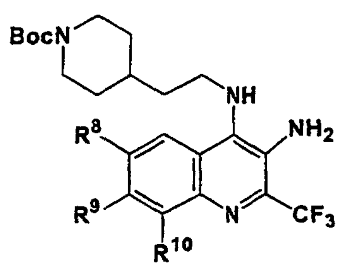

| Reference example | R 8 | R 9 | R10 | Physical properties (Recrystallization solvent) |
|---|---|---|---|---|
| 63 | Me | H | H | colorless crystals (iso-$Pr_2O$-n-Heptane) mp, 77-78°C<br>Elemental analysis for $C_{23}H_{31}F_3N_4O_2$<br>Calcd.%: C, 61.05; H, 6.91; N, 12.38<br>Found%: C, 61.09; H, 6.80; N, 12.43 |
| 64 | H | Cl | H | pale yellow crystals(iso-$Pr_2O$) mp, 126.5-127.5°C<br>Elemental analysis for $C_{22}H_{28}ClF_3N_4O_2$<br>Calcd.%: C, 55.87; H, 5.97; N, 11.85<br>Found%: C, 55.94; H, 5.91; N, 11.75 |
| 65 | H | F | H | pate yellow needles(iso-$Pr_2O$) mp, 98-99°C<br>Elemental analysis for $C_{22}H_{28}F_4N_4O_2$<br>Calcd.%: C, 57.89; H, 6.18; N, 12.27<br>Found%: C, 57.99; H, 6.39; N, 12.07 |
| 66 | H | Me | H | pale yellow crystals (iso-$Pr_2O$-n-Heptane) mp, 108-109°C<br>Elemental analysis for $C_{23}H_{31}F_3N_4O_2$<br>Calcd.%: C, 61.05; H, 6.91; N, 12.38<br>Found%: C, 61.01; H, 6.92; N, 12.13 |
| 67 | H | H | Me | colorless crystals (iso-$Pr_2O$-n-Heptane) mp, 105-106°C<br>Elemental analysis for $C_{23}H_{31}F_3N_4O_2$<br>Calcd.%: C, 61.05; H, 6.91; N, 12.38<br>Found%: C, 61.06; H, 6.94; N, 12.20 |

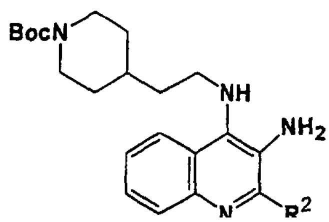

| Reference example | $-R^2$ | Physical properties (Recrystallization solvent) |
|---|---|---|
| 68 | 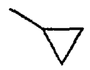 | pale brown needles<br>(AcOEt-n-Heptane)<br>mp,150.5-152°C<br>Elemental analysis for $C_{24}H_{34}N_4O_2$<br>Calcd.%: C, 70.21; H, 8.35; N, 13.65<br>Found%: C, 70.13; H, 8.22; N, 13.55 |
| 69 | -Ph | pale yellow crystals<br>(AcOEt-n-Heptane)<br>mp,113.5-115°C<br>Elemental analysis for $C_{27}H_{34}N_4O_2$<br>Calcd.%: C, 72.62; H, 7.67; N, 12.55<br>Found%: C, 72.69; H, 7.64; N, 12.53 |
| 70 | 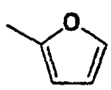 | yellowish brown crystals(iso-$Pr_2O$)<br>mp,106.5-108°C<br>Elemental analysis for $C_{25}H_{32}N_4O_3$<br>Calcd.%: C, 68.78; H, 7.39; N, 12.83<br>Found%: C, 68.68; H, 7.18; N, 12.76 |
| 71 | 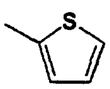 | yellow prisms(AcOEt)<br>mp,129.5-131°C<br>Elemental analysis for $C_{25}H_{32}N_4O_2S$<br>Calcd.%: C, 66.34; H, 7.13; N, 12.38<br>Found%: C, 66.25; H, 6.92; N, 12.29 |

Reference example 72

1-[2-[4-(1-tert-Butoxycarbonyl)piperidyl]ethyl]-2-phenyl-1H-imidazo[4,5-c]quinoline 5-oxide

To a solution of 3.67 g of tert-butyl 4-[2-(2-phenyl-lH-imidazo[4,5-c]quinolin-1-yl)ethyl]-1-piperidinecarboxylate in 110ml of 1,2-dichloroethane, 5.95 g of metachloroperbenzoic acid was added little by little, and the mixture was stirred at room temperature for 30 minutes. The reaction mixture was adjusted to pH 10 with 10 % aqueous solution of sodium hydroxide and extracted with 1,2-dichloroethane. The extract was washed successively with 10 % aqueous solution of sodium hydroxide and saturated brine, and dried, and the solvent was evaporated to give 4.28 g of a brown solid. The residue was washed successively with ethyl acetate and diethyl ether to give 2.46 g of colorless crystals.
NMR spectrum δ (DMSO-$d_6$) ppm:0.80-0.92 (2H,m), 1.22-1.32 (3H,m), 1.36(9H,s), 1.73 (2H,q,J=7.5Hz), 2.52 (2H,t, J=13Hz), 3.76 (2H,d,J=13Hz), 4.70 (2H,t,J=7.5Hz), 7.60-7.67 (3H,m), 7.75-7.80 (2H,m), 7.84 (1H,t,J=8Hz) 7.92 (1H, t,J=8Hz), 8.43 (1H,d,J=8Hz), 8.86 (1H,d,J=8Hz), 9.07 (1H,s)
IR spectrum ν (KBr)$cm^{-1}$:1696,1166
Mass spectrum m/z: 472($M^+$)

Example 1

tert-Butyl 4-[2-(2-phenyl-4-trifluoromethyl-1H-imidazo[4,5-c]quinolin-1-yl)ethyl]-1piperidinecarboxylate

A solution of 0.70 g of tert-butyl 4-[2-[(3-amino-2-trifluoromethylquinolin4-yl)amino]ethyl]-1-piperidinecarboxylate, 0.25 g of benzaldehyde and 0.04 g of 2,3-dichloro-5,6-dicyano-1,4-benzoquinone in 5 ml of tetrahydrofuran was stirred at room temperature for 3 days. The reaction mixture was added with saturated aqueous solution of sodium hydrogencarbonate, and extracted with ethyl acetate. The extract was washed successively with saturated aqueous solution of sodium hydrogencarbonate and saturated brine, and dried, and the solvent was evaporated. The residue was washed with diisopropyl ether to give 0.51 g of crystals. Recrystallization from diisopropyl ether gave colorless crystals having the melting point of from 163 to 164°C.
Elemental analysis for $C_{29}H_{31}F_3N_4O_2 \cdot 1/4H_2O$
Calculated % C,65.83; H,6.00; N,10.59
Found % C,65.85; H,5.90; N,10.58

Example 2

tert-Butyl 4-[2-[2-(4-iodophenyl)-8-methyl-4-trifluoromethyl-1H-imidazo[4,5-c]quinolin-1-yl]ethyl]-1-piperidinecarboxylate

A solution of 2.00 g of tert-butyl 4-[2-[(3-amino-6-methyl-2-trifluoromethylquinolin-4-yl)amino]ethyl]-1-piperidinecarboxylate and 0.74 ml of triethylamine in 20 ml of toluene was heated at 70°C, and added with 1.41 g of 4-iodobenzoyl chloride, and the mixture was stirred at 70°C for 3 hours. An insoluble matter was filtered off with hot condition, and the filtrate was added with 0.08 g of p-toluenesulfonic acid monohydrate, and the mixture was stirred at 120°C for 1.5 hours. After the reaction, the solvent was evaporated, and the residue was purified by silica gel column chromatography using a mixture of ethyl acetate and n-heptane (1:3) as eluting solvent and washed with diisopropyl ether to give 2.20 g of colorless crystals. Recrystallization from a mixture of ethyl acetate and diisopropyl ether gave colorless crystals having the melting point of from 203 to 204°C.
Elemental analysis for $C_{30}H_{32}F_3IN_4O_2$
Calculated % C,54.22; H,4.85; N,8.43
Found % C,54.25; H,4.78; N,8.38

In accordance with the methods of Examples 1 and 2, the compounds of Examples 3 through 57 were obtained.

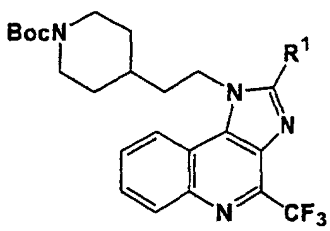

| Example | -$R^1$ | Physical properties (Recrystallization solvent) |
|---|---|---|
| 3 | 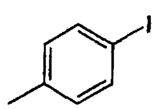 | colorless crystals(AcOEt-iso-$Pr_2O$)<br>mp,196-197°C<br>Elemental analysis for $C_{29}H_{30}F_3IN_4O_2$<br>Calcd.% :C, 53.55; H, 4.65; N, 8.61<br>Found% :C, 53.48; H, 4.73; N, 8.50 |
| 4 | 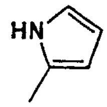 | colorless crystals(AcOEt)<br>mp,207.5-209.5°C(decomposition)<br>Elemental analysis for $C_{27}H_{30}F_3N_5O_2$<br>Calcd.% :C, 63.15; H, 5.89; N, 13.64<br>Found% :C, 62.95; H, 5.90; N, 13.59 |
| 5 | 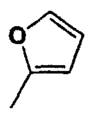 | pale yellow crystals(AcOEt)<br>mp,198.5-199.5°C<br>Elemental analysis for $C_{27}H_{29}F_3N_4O_3$<br>Calcd.% :C, 63.03; H, 5.68; N, 10.89<br>Found% :C, 62.91; H, 5.64; N, 10.87 |
| 6 | 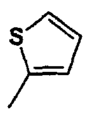 | colorless needles(2-PrOH)<br>mp,204.5-205.5°C<br>Elemental analysis for $C_{27}H_{29}F_3N_4O_2S$<br>Calcd.% :C, 61.12; H, 5.51; N, 10.56<br>Found% :C, 60.98; H, 5.46; N, 10.43 |

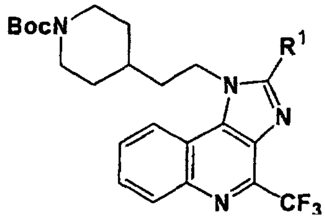

| Example | -$R^1$ | Physical properties (Recrystallization solvent) |
|---|---|---|
| 7 | 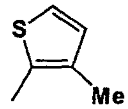 | pale brown needles(2-PrOH)<br>mp,166.5-167.5°C<br>Elemental analysis for $C_{28}H_{31}F_3N_4O_2S$<br>Calcd.% :C, 61.75; H, 5.74; N, 10.29<br>Found% :C, 61.50; H, 5.62; N, 10.12 |
| 8 | 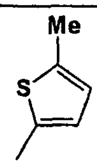 | colorless crystals(EtOH)<br>mp,217-218°C<br>Elemental analysis for $C_{28}H_{31}F_3N_4O_2S$<br>Calcd.% :C, 61.75; H, 5.74; N, 10.29<br>Found% :C, 61.68; H, 5.67; N, 10.27 |
| 9 | 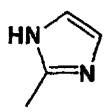 | pale brown needles(AcOEt-iso-$Pr_2O$)<br>mp,213.5-215°C<br>Elemental analysis for $C_{28}H_{29}F_3N_6O_2$<br>Calcd.% :C, 60.69; H, 5.68; N, 16.33<br>Found% :C, 60.62; H, 5.69; N, 16.23 |
| 10 | 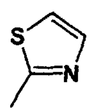 | colorless needles(AcOEt)<br>mp,203-205°C<br>Elemental analysis for $C_{26}H_{28}F_3N_5O_2S$<br>Calcd.% :C, 58.74; H, 5.31; N, 13.17<br>Found% :C, 58.62; H, 5.30; N, 13.04 |
| 11 | 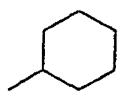 | pale brown crystals(AcOEt-iso-$Pr_2O$)<br>mp,189-191.5°C<br>Elemental analysis for $C_{29}H_{37}F_3N_4O_2$<br>Calcd.% :C, 65.64; H, 7.03; N, 10.56<br>Found% :C, 65.42; H, 6.93; N, 10.46 |

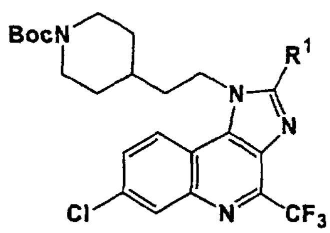

| Example | $-R^1$ | Physical properties (Recrystallization solvent) |
|---|---|---|
| 12 | -Ph | colorless crystals(AcOEt-iso-$Pr_2O$)<br>mp, 190-190.5°C<br>Elemental analysis for $C_{29}H_{30}ClF_3N_4O_2$<br>Calcd.% :C, 62.31; H, 5.41; N, 10.02<br>Found% :C, 62.23; H, 5.33; N, 10.00 |
| 13 | 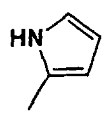 | pale brown crystals(AcOEt)<br>mp, 211.5-212.5°C(decomposition)<br>Elemental analysis for $C_{27}H_{29}ClF_3N_5O_2$<br>Calcd.% :C, 59.18; H, 5.33; N, 12.78<br>Found% :C, 59.17; H, 5.30; N, 12.66 |
| 14 | 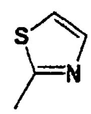 | pale yellow crystals(AcOEt)<br>mp, 206-207°C<br>Elemental analysis for $C_{26}H_{27}ClF_3N_5O_2S$<br>Calcd.% :C, 55.17; H, 4.81; N, 12.37<br>Found% :C, 55.25; H, 4.97; N, 12.44 |
| 15 | 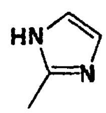 | colorless crystals(AcOEt-iso-$Pr_2O$)<br>mp, 222.5-230°C(decomposition)<br>Elemental analysis for $C_{26}H_{28}ClF_3N_6O_2$<br>Calcd.% :C, 56.88; H, 5.14; N, 15.31<br>Found% :C, 57.01; H, 5.10; N, 15.29 |
| 16 | 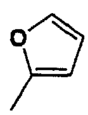 | colorless crystals(AcOEt)<br>mp, 199-200°C<br>Elemental analysis for $C_{27}H_{28}ClF_3N_4O_3$<br>Calcd.% :C, 59.07; H, 5.14; N, 10.21<br>Found% :C, 59.03; H, 5.19; N, 10.20 |
| 17 | 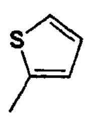 | pale brown crystals(AcOEt)<br>mp, 191.5-192.5°C<br>Elemental analysis for $C_{27}H_{28}ClF_3N_4O_2S$<br>Calcd.% :C, 57.39; H, 4.99; N, 9.92<br>Found% :C, 57.34; H, 5.01; N, 9.92 |
| 18 | 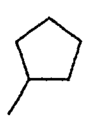 | colorless crystals(AcOEt-iso-$Pr_2O$)<br>mp, 189-190°C<br>Elemental analysis for $C_{28}H_{34}ClF_3N_4O_2$<br>Calcd.% :C, 61.03; H, 6.22; N, 10.17<br>Found% :C, 61.07; H, 6.06; N, 9.97 |

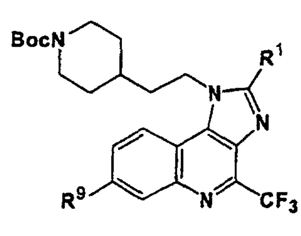

| Example | $-R^1$ | $R^9$ | Physical properties (Recrystallization solvent) |
| --- | --- | --- | --- |
| 19 | 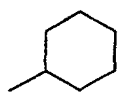 | Cl | yellowish orange crystals (AcOEt-iso-$Pr_2O$)<br>mp, 185-186°C<br>Elemental analysis for $C_{29}H_{36}ClF_3N_4O_2$<br>Calcd.% :C, 61.64; H, 6.42; N, 9.92<br>Found% :C, 61.57; H, 6.49; N, 9.94 |
| 20 | -Ph | F | pale yellow plates(AcOEt-iso-$Pr_2O$)<br>mp, 193-194°C<br>Elemental analysis for<br>$C_{29}H_{30}F_4N_4O_2 \cdot 1/4H_2O$<br>Calcd.% :C, 63.67; H, 5.62; N, 10.24<br>Found% :C, 63.51; H, 5.69; N, 10.15 |
| 21 | 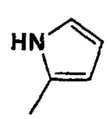 | F | pale brown needles(AcOEt-iso-$Pr_2O$)<br>mp, 220-221°C<br>Elemental analysis for $C_{27}H_{29}F_4N_5O_2$<br>Calcd.% :C, 61.01; H, 5.50; N, 13.18<br>Found% :C, 60.98; H, 5.46; N, 13.16 |
| 22 | 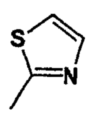 | F | colorless needles(AcOEt)<br>mp, 222-223°C<br>Elemental analysis for $C_{26}H_{27}F_4N_5O_2S$<br>Calcd.% :C, 56.82; H, 4.95; N, 12.74<br>Found% :C, 56.91; H, 5.05; N, 12.63 |
| 23 | 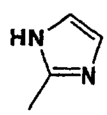 | F | colorless plates(AcOEt-iso-$Pr_2O$)<br>mp, 203-204°C<br>Elemental analysis for $C_{26}H_{28}F_4N_6O_2$<br>Calcd.% :C, 58.64; H, 5.30; N, 15.78<br>Found% :C, 58.33; H, 5.45; N, 15.63 |
| 24 | 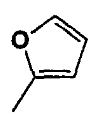 | F | pale yellow plates(AcOEt-iso-$Pr_2O$)<br>mp, 209-210°C<br>Elemental analysis for $C_{27}H_{28}F_4N_4O_3$<br>Calcd.% :C, 60.90; H, 5.30; N, 10.52<br>Found% :C, 60.65; H, 5.24; N, 10.42 |
| 25 | 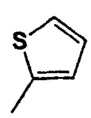 | F | pale yellow needles(AcOEt-iso-$Pr_2O$)<br>mp, 215-216°C<br>Elemental analysis for $C_{27}H_{28}F_4N_4O_2S$<br>Calcd.% :C, 59.11; H, 5.14; N, 10.21<br>Found% :C, 59.14; H, 5.33; N, 10.14 |

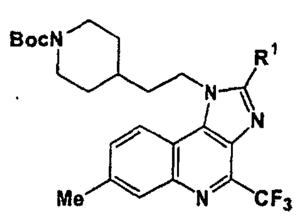

| Example | $-R^1$ | Physical properties (Recrystallization solvent) |
|---|---|---|
| 26 | -Ph | colorless crystals(AcOEt-iso-$Pr_2O$)<br>mp, 169-170°C<br>Elemental analysis for $C_{30}H_{33}F_3N_4O_2$<br>Calcd.% :C, 66.90; H, 6.18; N, 10.40<br>Found% :C, 66.93; H, 6.19; N, 10.17 |
| 27 | 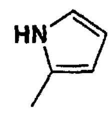 | pale orange crystals(AcOEt-iso-$Pr_2O$)<br>mp, 215-216°C(decomposition)<br>Elemental analysis for $C_{28}H_{32}F_3N_5O_2$<br>Calcd.% :C, 63.74; H, 6.11; N, 13.27<br>Found% :C, 63.54; H, 6.08; N, 13.04 |
| 28 | 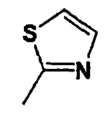 | pale yellow needles(AcOEt)<br>mp, 215-216°C<br>Elemental analysis for $C_{27}H_{30}F_3N_5O_2S$<br>Calcd.% :C, 59.43; H, 5.54; N, 12.84<br>Found%: C, 59.35; H, 5.65; N, 12.64 |
| 29 | 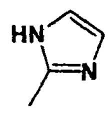 | pale yellow needles(AcOEt)<br>mp, 232-233°C<br>Elemental analysis for $C_{27}H_{31}F_3N_6O_2\cdot 3/4H_2O$<br>Calcd.% :C, 59.82; H, 6.04; N, 15.50<br>Found%: C, 59.74; H, 5.99; N, 15.80 |
| 30 | 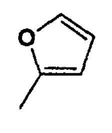 | pale orange needles(AcOEt)<br>mp, 217-218°C<br>Elemental analysis for $C_{28}H_{31}F_3N_4O_3$<br>Calcd.% :C, 63.62; H, 5.91; N, 10.60<br>Found% :C, 63.59; H, 5.98; N, 10.52 |
| 31 | 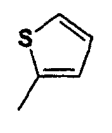 | pale yellow needles(AcOEt)<br>mp, 218-219°C<br>Elemental analysis for $C_{28}H_{31}F_3N_4O_2S$<br>Calcd.% :C, 61.75; H, 5.74; N, 10.29<br>Found% :C, 61.48; H, 5.73; N, 10.12 |
| 32 | 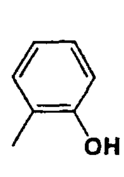 | colorless crystals(AcOEt-MeOH)<br>NMR $\delta$ (DMSO-$d_6$)ppm:0.77(2H,q,J=12Hz),1.15-1.28(3H,m),1.34(9H,s),1.62(2H,q,J=7Hz),2.40-2.50(2H,m),2.58(3H,s),3.69(2H,d,J=12Hz),4.67(2H,t,J=7Hz),6.58(1H,t,J=8Hz),6.79(1H,d,J=8Hz),7.19(1H,t,J=8Hz),7.30(1H,d,J=8Hz),7.67(1H,dd,J=7.5,1Hz),8.07(1H,d,J=1Hz),8.33(1H,d,J=7.5Hz)<br>IR $\nu$ (KBr)$cm^{-1}$: 3400, 1696<br>MS m/z: 553($M^+$-1) |

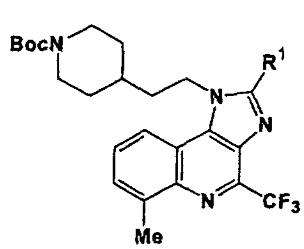

| Example | $-R^1$ | Physical properties (Recrystallization solvent) |
|---|---|---|
| 33 | -Ph | colorless crystals(AcOEt-iso-$Pr_2O$)<br>mp, 171-172°C<br>Elemental analysis for $C_{30}H_{33}F_3N_4O_2$<br>Calcd.% :C, 66.90; H, 6.18; N, 10.40<br>Found% :C, 66.89; H, 6.18; N, 10.18 |
| 34 | 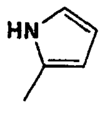 | colorless crystals(AcOEt-iso-$Pr_2O$)<br>mp, 189-190°C<br>Elemental analysis for $C_{28}H_{32}F_3N_5O_2$<br>Calcd.% :C, 63.74; H, 6.11; N, 13.27<br>Found% :C, 63.74; H, 6.05; N, 13.11 |
| 35 | 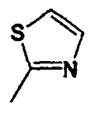 | pale yellow amorphous solid<br>NMR $\delta$ ($CDCl_3$)ppm:1.20-1.39(2H,m),1.47(9H,s),1.67-1.90(3H,m),2.03(2H,q,J=7.5Hz),2.65-2.83(2H,m),2.93(3H,s),4.00-4.26(2H,m),5.23-5.55(2H,m),7.57(1H,d,J=3.5Hz),7.62-7.68(2H,m),8.00(1H,d,J=3.5Hz),8.12-8.18(1H,m)<br>IR $\nu$ (KBr)$cm^{-1}$:1694<br>MS m/z:546($M^+$+1) |
| 36 | 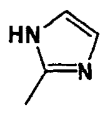 | pale yellow amorphous solid<br>NMR $\delta$ ($CDCl_3$)ppm:1.20-1.39(2H,m),1.47(9H,s),1.68-1.93(3H,m),2.03(2H,q,J=7.5Hz),2.65-2.83(2H,m),2.90(3H,s),4.00-4.28(2H,m),5.25-5.80(2H,m),7.20(1H,s),7.31(1H,s),7.61-7.69(2H,m),8.15-8.21(1H,m),11.71(1H,brs)<br>IR $\nu$ (KBr)$cm^{-1}$:1694<br>MS m/z:529($M^+$+1) |
| 37 | 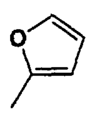 | pale yellow amorphous solid<br>NMR $\delta$ ($CDCl_3$)ppm:1.20-1.36(2H,m),1.47(9H,s),1.54-1.84(3H,m),2.03(2H,q,J=7.5Hz),2.62-2.81(2H,m),2.92(3H,s),3.97-4.30(2H,m),4.90(2H,t,J=7.5Hz),6.66(1H,dd,J=3,2Hz),7.30(1H,dd,J=3,1Hz),7.60-7.68(3H,m),8.06-8.11(1H,m)<br>IR $\nu$ (KBr)$cm^{-1}$:1694<br>MS m/z:529($M^+$+1) |
| 38 | 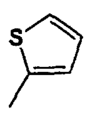 | pale yellow amorphous solid<br>NMR $\delta$ ($CDCl_3$)ppm:1.15-1.31(2H,m),1.46(9H,s),1.52-1.72(3H,m),1.97(2H,q,J=8Hz),2.60-2.78(2H,m),2.93(3H,s),3.98-4.23(2H,m),4.79(2H,t,J=8Hz),7.24(1H,dd,J=5,3.5Hz),7.57-7.67(4H,m),8.03-8.08(1H,m)<br>IR $\nu$ (KBr)$cm^{-1}$:1692<br>MS m/z:545($M^+$+1) |

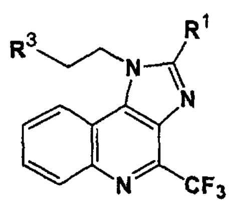

| Example | $-R^1$ | $R^3$- | Physical properties (Recrystallization solvent) |
|---|---|---|---|
| 39 | -Ph | 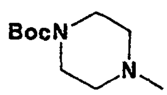 | colorless crystals(AcOEt)<br>mp, 204-205°C<br>Elemental analysis for $C_{28}H_{30}F_3N_5O_2$<br>Calcd.% :C, 63.99; H, 5.75; N, 13.33<br>Found% :C, 63.77; H, 5.68; N, 13.35 |
| 40 | 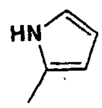 | 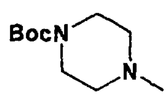 | pale brown crystals<br>NMR $\delta$ (DMSO-$d_6$)ppm:1.37(9H,s),2.32(4H,t,J=5Hz),2.90(2H,t,J=6.5Hz),3.17(4H,t,J=5Hz),5.02(2H,t,J=6.5Hz),6.34(1H,dd,J=6,2.5Hz),6.87(1H,brs),7.10(1H,brs),7.83(1H,t,J=8Hz),7.88(1H,t,J=8Hz),8.28(1H,d,J=8Hz),8.55(1H,d,J=8Hz),11.73(1H,brs)<br>IR $\nu$ (KBr)$cm^{-1}$:3460,1690<br>MS m/z:513($M^+$-1) |
| 41 | -Ph | 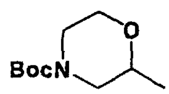 | colorless plates(EtOH)<br>mp,189-190°C<br>Elemental analysis for $C_{28}H_{29}F_3N_4O_3$<br>Calcd.% :C, 63.87; H, 5.55; N, 10.64<br>Found% :C, 63.87; H, 5.50; N, 10.68 |
| 42 | 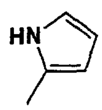 | 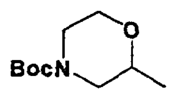 | pale orange needles(MeOH)<br>mp,219-220°C<br>Elemental analysis for $C_{26}H_{28}F_3N_5O_3$<br>Calcd.% :C, 60.57; H, 5.47; N, 13.58<br>Found% :C, 60.43; H, 5.34; N, 13.57 |
| 43 | -Ph | 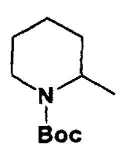 | pale yellowish brown crystals (AcOEt-iso-$Pr_2O$)<br>mp, 161-162°C<br>Elemental analysis for $C_{29}H_{31}F_3N_4O_2$<br>Calcd.% :C, 66.40; H, 5.96; N, 10.68<br>Found% :C, 66.51; H, 6.09; N, 10.70 |
| 44 | 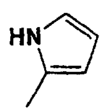 | 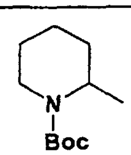 | colorless crystals(AcOEt-iso-$Pr_2O$)<br>mp, 183.5-184.5°C<br>Elemental analysis for $C_{27}H_{30}F_3N_5O_2$<br>Calcd.% :C, 63.15; H, 5.89; N, 13.64<br>Found% :C, 63.00; H, 5.80; N, 13.56 |

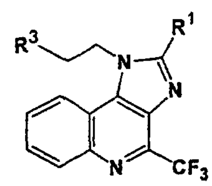

| Example | $-R^1$ | $R^3$- | Physical properties [salt] (Recrystallization solvent) |
|---|---|---|---|
| 45 | 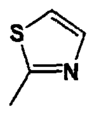 | 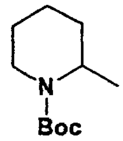 | colorless crystals(AcOEt-iso-$Pr_2O$)<br>mp, 168.5-169.5°C<br>Elemental analysis for $C_{28}H_{28}F_3N_5O_2S$<br>Calcd.% :C, 58.74; H, 5.31; N, 13.17<br>Found% :C, 58.90; H, 5.32; N, 13.18 |
| 46 | -Ph | 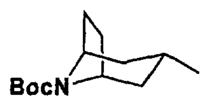 | colorless needles(AcOEt-iso-$Pr_2O$)<br>mp, 219-221°C<br>Elemental analysis for $C_{31}H_{33}F_3N_4O_2$<br>Calcd.% :C, 67.62; H, 6.04; N, 10.18<br>Found% :C, 67.52; H, 6.16; N, 10.11 |
| 47 | 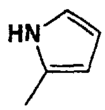 | 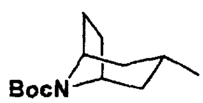 | pale orange plates(AcOEt-iso-$Pr_2O$)<br>mp, 158-160°C<br>Elemental analysis for<br>$C_{29}H_{32}F_3N_5O_2 \cdot 1/4H_2O$<br>Calcd.% :C, 64.02; H, 6.02; N, 12.87<br>Found% :C, 63.98; H, 5.94; N, 12.72 |
| 48 | -Ph | BocHN- | colorless crystals(AcOEt)<br>mp, 222.5-224°C<br>Elemental analysis for $C_{24}H_{23}F_3N_4O_2$<br>Calcd.% :C, 63.15; H, 5.08; N, 12.27<br>Found% :C, 63.16; H, 5.06; N, 12.26 |
| 49 | 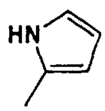 | BocHN- | colorless crystals(AcOEt-iso-$Pr_2O$)<br>mp, 166-169°C (decomposition)<br>Elemental analysis for $C_{22}H_{22}F_3N_5O_2$<br>Calcd.% :C, 59.32; H, 4.98; N, 15.72<br>Found% :C, 59.44; H, 5.02; N, 15.42 |
| 50 | -Ph | $Me_2N$- | pale brown crystals[fumarate](EtOH)<br>mp,205.5-207.5°C(decomposition)<br>Elemental analysis for<br>$C_{21}H_{19}F_3N_4 \cdot 1/2C_4H_4O_4$<br>Calcd.% :C, 62.44; H, 4.78; N, 12.66<br>Found% :C, 62.28; H, 4.97; N, 12.69 |
| 51 | 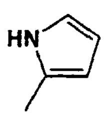 | $Me_2N$- | pale brown crystals[fumarate](EtOH)<br>mp,228-230°C(decomposition)<br>Elemental analysis for<br>$C_{19}H_{18}F_3N_5 \cdot 1/2C_4H_4O_4$<br>Calcd.% :C, 58.47; H, 4.67; N, 16.23<br>Found% :C, 58.31; H, 4.72; N, 16.14 |

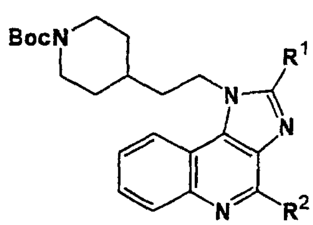

| Example | $-R^1$ | $-R^2$ | Physical properties (Recrystallization solvent) |
|---|---|---|---|
| 52 | -Ph | 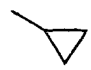 | pale brown crystals(AcOEt-iso-$Pr_2O$)<br>mp,150-151°C<br>Elemental analysis for $C_{31}H_{36}N_4O_2$<br>Calcd.% :C, 74.97; H, 7.31; N, 11.28<br>Found% :C, 74.62; H, 7.25; N, 11.09 |
| 53 | 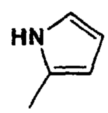 | 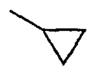 | colorless needles(AcOEt)<br>mp,196-197.5°C<br>Elemental analysis for $C_{29}H_{35}N_5O_2$<br>Calcd.% :C, 71.72; H, 7.26; N, 14.42<br>Found% :C, 71.57; H, 7.27; N, 14.38 |
| 54 | -Ph | -Ph | colorless needles(AcOEt)<br>mp,181-182°C<br>Elemental analysis for $C_{34}H_{36}N_4O_2$<br>Calcd.% :C, 76.66; H, 6.81; N, 10.52<br>Found% :C, 76.49; H, 6.80; N, 10.50 |
| 55 | 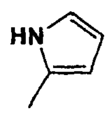 | -Ph | brown crystals(EtOH)<br>mp,198.5-200°C<br>Elemental analysis for<br>$C_{32}H_{35}N_5O_2 \cdot 1/4H_2O$<br>Calcd.% :C, 73.05; H, 6.80; N, 13.31<br>Found% :C, 73.04; H, 6.71; N, 13.27 |
| 56 | -Ph | 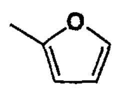 | colorless crystals(MeOH)<br>mp,214.5-216°C<br>Elemental analysis for $C_{32}H_{34}N_4O_3$<br>Calcd.% :C, 73.54; H, 6.56; N, 10.72<br>Found% :C, 73.43; H, 6.54; N, 10.66 |
| 57 | -Ph | 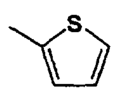 | colorless crystals(MeOH-AcOEt)<br>mp,221.5-222.5°C<br>Elemental analysis for $C_{32}H_{34}N_4O_2S$<br>Calcd.% :C, 71.35; H, 6.36; N, 10.40<br>Found% :C, 71.21; H, 6.27; N, 10.34 |

Example 58

tert-Butyl 4-[2-(4-mercapto-2-phenyl-1H-imidazo[4,5-c]quinolin-1-yl)ethyl]-1-piperidinecarboxylate

A mixture of 1.00 g of tert-butyl 4-[2-(4-chloro-2-phenyl-1H-imidazo[4,5-c]quinolin-1-yl)ethyl]-1-piperidinecarboxylate, 0.62 g of thiourea and 20 ml of ethanol was heated with evaporating ethanol at 100°C for 2 hours. The reaction mixture was left to cool, and added with 1.4 ml of triethylamine. The precipitated crystals were collected by filtration, and washed successively with water and methanol to give 0.75 g of pale brown crystals. The crystals were purified by silica gel column chromatography using ethyl acetate as eluting solvent to give 0.57 g of colorless crystals. Recrystallization from a mixture of ethyl acetate and methanol gave colorless crystals having the melting point of from 253 to 258°C(decomposition).
Elemental analysis for $C_{28}H_{32}N_4O_2S \cdot 1/4H_2O$
Calculated % C,68.19; H,6.64; N,11.36
Found % C,68.34; H,6.52; N,11.37

Example 59

tert-Butyl 4-[2-(4-cyano-2-phenyl-1H-imidazo[4,5-c]quinolin-1-yl)ethyl]-1-piperidinecarboxylate

To a solution of 0.37 g of 1-[2-[4-(1-tert-butoxycarbonyl)piperidyl]ethyl]-2-phenyl-lH-imidazo[4,5-c]quinoline

5-oxide and 0.13 ml of cyanotrimethylsilane in 7.4 ml of tetrahydrofuran, a solution of 0.26 ml of 1,8-diazabicyclo[5.4.0]-7-undecene in 2.6 ml of tetrahydrofuran was added dropwise at room temperature, and the mixture was refluxed for 2 hours. After the reaction, the solvent was evaporated, and the residue was purified by silica gel column chromatography using a mixture of ethyl acetate and n-heptane (1:1) as eluting solvent to give 0.24 g of colorless crystals. Recrystallization from a mixture of methanol and 2-propanol gave colorless crystals having the melting point of from 215.5 to 217°C.
Elemental analysis for $C_{29}H_{31}N_5O_2$
Calculated % C,72.33; H,6.49; N,14.54
Found % C,72.21; H,6.42; N,14.51

Example 60

1-[2-[4-(1-tert-Butoxycarbonyl)piperidyl]ethyl]-2-phenyl-1H-imidazo[4,5-c]quinoline-4-carboxylic acid

A mixture of 0.80 g of tert-butyl 4-[2-(4-cyano-2-phenyl-1H-imidazo[4,5-c]quinolin-1-yl)ethyl]-1-piperidinecarboxylate, 8 ml of 10% aqueous solution of sodium hydroxide and 16 ml of ethanol was refluxed for 4 hours. The reaction mixture was left to cool, and neutralized with 10% hydrochloric acid, and adjusted to pH 3-4 with 10% aqueous solution of citric acid, and the solvent was evaporated. The precipitated crystals were washed with water to give 0.72 g of colorless crystals. Recrystallization from methanol gave colorless crystals having the melting point of from 201.5 to 204°C.
Elemental analysis for $C_{29}H_{32}N_4O_4 \cdot 1/4H_2O$
Calculated % C,68.96; H,6.49; N,11.09
Found % C,69.03; H,6.27; N,11.03

Example 61

2-Phenyl-1-[2-(4-piperidyl)ethyl]-4-trifluoromethyl-1H-imidazo[4,5-c]quinoline

To a solution of 0.40 g of tert-butyl 4-[2-(2-phenyl-4-trifluoromethyl-1H-imidazo[4,5-c]quinolin-1-yl)ethyl]-1-piperidinecarboxylate in 3 ml of 1,2-dichloroethane, 1 ml of trifluoroacetic acid was added, and the mixture was stirred at room temperature for 30 minutes. The reaction mixture was poured into aqueous solution of potassium carbonate, and extracted with 1,2-dichloroethane. The extract was dried, and the solvent was evaporated. The residue was washed with diisopropyl ether to give 0.32 g of pale brown crystals. Recrystallization from ethyl acetate gave pale brown crystals having the melting point of from 172.5 to 173.5°C.
Elemental analysis for $C_{24}H_{23}F_3N_4$
Calculated % C,67.91; H,5.46; N,13.20
Found % C,67.73; H,5.42; N,13.07
In accordance with the method of Example 61, the compounds of Examples 62 through 118 were obtained.

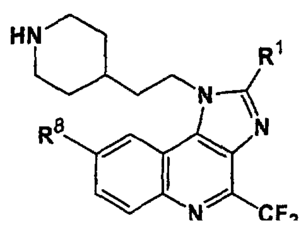

| Example | $-R^1$ | $R^8$ | Physical properties [salt] (Recrystallization solvent) |
|---|---|---|---|
| 62 | 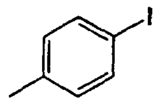 | H | colorless crystals [trifluoroacetate] (EtOH-AcOEt)<br>mp,198.5-200.5°C (decomposition)<br>Elemental analysis for $C_{24}H_{22}F_3IN_4 \cdot CF_3CO_2H$<br>Calcd.% :C, 47.00; H, 3.49; N, 8.43<br>Found% :C, 46.74; H, 3.48; N, 8.43 |
| 63 | 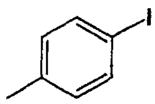 | Me | colorless crystals [trifluoroacetate] (EtOH-AcOEt)<br>mp,222.5-223.5°C (decomposition)<br>Elemental analysis for $C_{25}H_{24}F_3IN_4 \cdot CF_3CO_2H$<br>Calcd.% :C, 47.80; H, 3.71; N, 8.26<br>Found% :C, 47.65; H, 3.73; N, 8.35 |
| 64 | 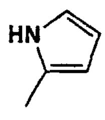 | H | pale brown crystals(MeOH)<br>mp,280-282.5°C (decomposition)<br>Elemental analysis for $C_{22}H_{22}F_3N_5$<br>Calcd.% :C, 63.91; H, 5.36; N, 16.94<br>Found% :C, 63.72; H, 5.36; N, 17.03 |
| 65 | 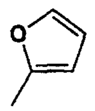 | H | brown crystals[trifluoroacetate] (AcOEt)<br>mp,133.5-136.5°C (decomposition)<br>Elemental analysis for<br>$C_{22}H_{21}F_3N_4O \cdot CF_3CO_2H \cdot 1/2H_2O$<br>Calcd.% :C, 53.63; H, 4.31; N, 10.42<br>Found% :C, 53.51; H, 4.29; N, 10.39 |
| 66 | 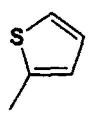 | H | pale brown needles[trifluoroacetate] (EtOH)<br>mp,237-239°C<br>Elemental analysis for<br>$C_{22}H_{21}F_3N_4S \cdot CF_3CO_2H \cdot 1/4H_2O$<br>Calcd.% :C, 52.50; H, 4.13; N, 10.20<br>Found% :C, 52.46; H, 4.19; N, 10.27 |

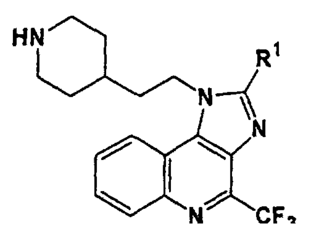

| Example | $-R^1$ | Physical properties [salt] (Recrystallization solvent) |
|---|---|---|
| 67 | 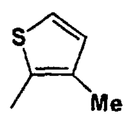 | pale brown needles[trifluoroacetate](2-PrOH)<br>mp,159-161℃<br>Elemental analysis for<br>$C_{23}H_{23}F_3N_4S \cdot CF_3CO_2H \cdot 1/4H_2O$<br>Calcd.% :C, 53.33; H, 4.39; N, 9.95<br>Found% :C, 53.19; H, 4.28; N, 9.95 |
| 68 | 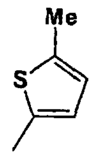 | colorless crystals[trifluoroacetate] (EtOH)<br>mp,208.5-211℃(decomposition)<br>$C_{23}H_{23}F_3N_4S \cdot CF_3CO_2H \cdot 1/4H_2O$<br>Calcd.% :C, 53.33; H, 4.39; N, 9.95<br>Found% :C, 53.30; H, 4.34; N, 10.03 |
| 69 | 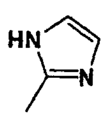 | colorless crystals[trifluoroacetate]<br>(MeOH-2-PrOH)<br>mp,283-284℃<br>Elemental analysis for<br>$C_{21}H_{21}F_3N_6 \cdot CF_3CO_2H \cdot H_2O$<br>Calcd.% :C, 50.55; H, 4.43; N, 15.38<br>Found% :C, 50.43; H, 4.31; N, 15.41 |
| 70 | 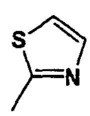 | pale brown needles(AcOEt)<br>mp,199-200℃<br>Elemental analysis for $C_{21}H_{20}F_3N_5S$<br>Calcd.% :C, 58.46; H, 4.67; N, 16.23<br>Found% :C, 58.28; H, 4.72; N, 16.05 |
| 71 | 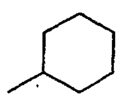 | colorless crystals[trifluoroacetate]<br>(AcOEt-EtOH)<br>mp,182.5-184℃<br>Elemental analysis for $C_{24}H_{29}F_3N_4 \cdot CF_3CO_2H$<br>Calcd.% :C, 57.35; H, 5.55; N, 10.29<br>Found% :C, 57.18; H, 5.49; N, 10.33 |

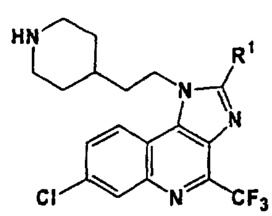

| Example | $-R^1$ | Physical properties [salt] (Recrystallization solvent) |
|---|---|---|
| 72 | -Ph | colorless crystals[trifluoroacetate](MeOH)<br>mp, 241-242.5°C (decomposition)<br>Elemental analysis for $C_{24}H_{22}ClF_3N_4 \cdot CF_3CO_2H$<br>Calcd.% :C, 54.51; H, 4.05; N, 9.78<br>Found% :C, 54.53; H, 4.14; N, 9.93 |
| 73 | 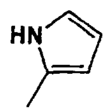 | pale brown crystals[trifluoroacetate](MeOH)<br>mp, 244-246°C (decomposition)<br>Elemental analysis for<br>$C_{22}H_{21}ClF_3N_5 \cdot CF_3CO_2H \cdot 3/4H_2O$<br>Calcd.% :C, 50.10; H, 4.12; N, 12.17<br>Found% :C, 50.23; H, 4.07; N, 12.36 |
| 74 | 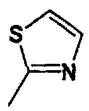 | pale brown crystals(MeOH)<br>mp, 232.5-234°C (decomposition)<br>Elemental analysis for $C_{21}H_{19}ClF_3N_5S$<br>Calcd.% :C, 54.13; H, 4.11; N, 15.03<br>Found% :C, 54.20; H, 4.24; N, 15.04 |
| 75 | 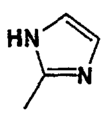 | colorless crystals[trifluoroacetate](MeOH)<br>mp, 290-295°C (decomposition)<br>Elemental analysis for $C_{21}H_{20}ClF_3N_6 \cdot CF_3CO_2H$<br>Calcd.% :C, 49.08; H, 3.76; N, 14.93<br>Found% :C, 49.11; H, 3.80; N, 14.83 |
| 76 | 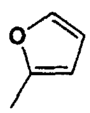 | colorless crystals[trifluoroacetate] (MeOH-AcOEt)<br>mp, 219-221°C (decomposition)<br>Elemental analysis for $C_{22}H_{20}ClF_3N_4O \cdot CF_3CO_2H$<br>Calcd.% :C, 51.21; H, 3.76; N, 9.95<br>Found% :C, 51.17; H, 3.75; N, 10.04 |
| 77 | 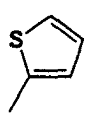 | colorless crystals[trifluoroacetate] (MeOH-AcOEt)<br>mp, 217-219°C (decomposition)<br>Elemental analysis for<br>$C_{22}H_{20}ClF_3N_4S \cdot CF_3CO_2H \cdot 1/2H_2O$<br>Calcd.% :C, 49.03; H, 3.77; N, 9.53<br>Found% :C, 49.03; H, 3.71; N, 9.56 |
| 78 | 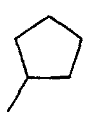 | colorless crystals[trifluoroacetate](MeOH)<br>mp, 246-248°C (decomposition)<br>Elemental analysis for $C_{23}H_{26}ClF_3N_4 \cdot CF_3CO_2H$<br>Calcd.% :C, 53.15; H, 4.82; N, 9.92<br>Found% :C, 53.27; H, 4.83; N, 9.94 |

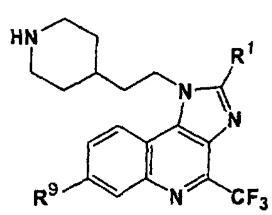

| Example | $-R^1$ | $R^9$ | Physical properties [salt] (Recrystallization solvent) |
|---|---|---|---|
| 79 | 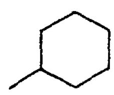 | Cl | pale brown crystals[trifluoroacetate] (EtOH)<br>mp, 236-237°C (decomposition)<br>Elemental analysis for $C_{24}H_{28}ClF_3N_4 \cdot CF_3CO_2H$<br>Calcd.% :C, 53.94; H, 5.05; N, 9.68<br>Found% :C, 53.67; H, 5.05; N, 9.71 |
| 80 | -Ph | F | pale yellow plates[trifluoroacetate](EtOH)<br>mp, 240-242°C (decomposition)<br>Elemental analysis for $C_{24}H_{22}F_4N_4 \cdot CF_3CO_2H$<br>Calcd.% :C, 56.12; H, 4.17; N, 10.07<br>Found% :C, 55.98; H, 4.25; N, 10.13 |
| 81 | 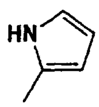 | F | pale gray needles[trifluoroacetate](EtOH)<br>mp, 252-254°C (decomposition)<br>Elemental analysis for<br>$C_{22}H_{21}F_4N_5 \cdot CF_3CO_2H \cdot 1/2H_2O$<br>Calcd.% :C, 51.99; H, 4.18; N, 12.63<br>Found% :C, 51.92; H, 4.26; N, 12.74 |
| 82 | 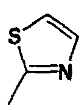 | F | pale yellow needles[trifluoroacetate] (EtOH)<br>mp, 236-237°C (decomposition)<br>Elemental analysis for $C_{21}H_{19}F_4N_5S \cdot CF_3CO_2H$<br>Calcd.% :C, 49.02; H, 3.58; N, 12.43<br>Found% :C, 48.72; H, 3.77; N, 12.36 |
| 83 | 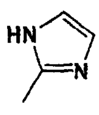 | F | colorless plates[trifluoroacetate](MeOH)<br>mp, 249-250°C (decomposition)<br>Elemental analysis for<br>$C_{21}H_{20}F_4N_6 \cdot CF_3CO_2H \cdot H_2O$<br>Calcd.% :C, 48.94; H, 4.11; N, 14.89<br>Found% :C, 48.80; H, 4.17; N, 15.01 |
| 84 | 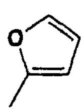 | F | pale yellow needles[trifluoroacetate] (EtOH)<br>mp, 239-241°C (decomposition)<br>Elemental analysis for<br>$C_{22}H_{20}F_4N_4O \cdot CF_3CO_2H \cdot 1/4H_2O$<br>Calcd.% :C, 52.32; H, 3.93; N, 10.17<br>Found% :C, 52.26; H, 3.82; N, 10.26 |
| 85 | 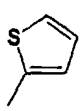 | F | pale yellow plates[trifluoroacetate](EtOH)<br>mp, 234-235°C (decomposition)<br>Elemental analysis for<br>$C_{22}H_{20}F_4N_4S \cdot CF_3CO_2H \cdot H_2O$<br>Calcd.% :C, 49.66; H, 3.99; N, 9.65<br>Found% :C, 49.71; H, 3.99; N, 9.67 |

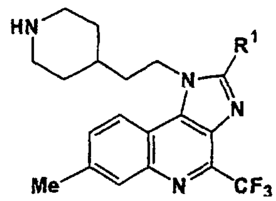

| Example | -$R^1$ | Physical properties [salt] (Recrystallization solvent) |
| --- | --- | --- |
| 86 | -Ph | colorless crystals[trifluoroacetate](EtOH)<br>mp, 234-235°C (decomposition)<br>Elemental analysis for $C_{25}H_{25}F_3N_4 \cdot CF_3CO_2H$<br>Calcd.% :C, 58.69; H, 4.74; N, 10.14<br>Found% :C, 58.44; H, 4.82; N, 10.01 |
| 87 | 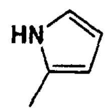 | grayish brown crystals[trifluoroacetate](MeOH)<br>mp, 280-284°C (decomposition)<br>Elemental analysis for $C_{23}H_{24}F_3N_5 \cdot CF_3CO_2H \cdot 3/4H_2O$<br>Calcd.% :C, 54.10; H, 4.81; N, 12.62<br>Found% :C, 54.14; H, 4.66; N, 12.70 |
| 88 | 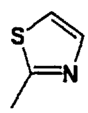 | pale yellowish brown needles[trifluoroacetate] (EtOH)<br>mp, 239-241°C (decomposition)<br>Elemental analysis for $C_{22}H_{22}F_3N_5S \cdot CF_3CO_2H \cdot 1/4H_2O$<br>Calcd.% :C, 51.11; H, 4.20; N, 12.42<br>Found% :C, 50.97; H, 4.01; N, 12.43 |
| 89 | 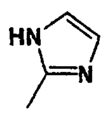 | pale yellow needles[trifluoroacetate](EtOH)<br>mp, 303-305°C (decomposition)<br>Elemental analysis for $C_{22}H_{23}F_3N_6 \cdot CF_3CO_2H \cdot H_2O$<br>Calcd.% :C, 51.43; H, 4.68; N, 14.99<br>Found% :C, 51.35; H, 4.39; N, 15.09 |
| 90 | 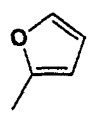 | pale yellowish brown needles[trifluoroacetate] (EtOH)<br>mp, 232-234°C (decomposition)<br>Elemental analysis for $C_{23}H_{23}F_3N_4O \cdot CF_3CO_2H \cdot H_2O$<br>Calcd.% :C, 53.57; H, 4.68; N, 10.00<br>Found% :C, 53.39; H, 4.65; N, 10.00 |
| 91 | 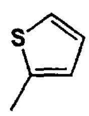 | pale yellow plates[trifluoroacetate](EtOH)<br>mp, 236-238°C (decomposition)<br>Elemental analysis for $C_{23}H_{23}F_3N_4S \cdot CF_3CO_2H \cdot 1/2H_2O$<br>Calcd.% :C, 52.91; H, 4.44; N, 9.87<br>Found% :C, 52.64; H, 4.27; N, 9.82 |
| 92 | 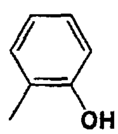 | colorless crystals[trifluoroacetate] (2-PrOH-AcOEt)<br>mp, 161-162°C<br>Elemental analysis for $C_{25}H_{25}F_3N_4O \cdot 5/4CF_3CO_2H$<br>Calcd.% :C, 55.32; H, 4.43; N, 9.38<br>Found% :C, 55.22; H, 4.28; N, 9.45 |

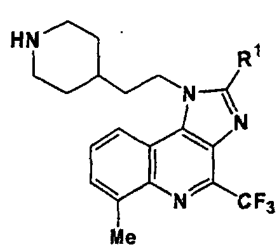

| Example | $-R^1$ | Physical properties [salt] (Recrystallization solvent) |
|---|---|---|
| 93 | -Ph | colorless crystals[trifluoroacetate](EtOH)<br>mp, 218-219°C(decomposition)<br>Elemental analysis for $C_{25}H_{25}F_3N_4 \cdot CF_3CO_2H$<br>Calcd.% :C, 58.69; H, 4.74; N, 10.14<br>Found% :C, 58.43; H, 4.82; N, 10.05 |
| 94 | 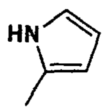 | colorless crystals[trifluoroacetate](MeOH)<br>mp, 233-234°C(decomposition)<br>Elemental analysis for<br>$C_{23}H_{24}F_3N_5 \cdot CF_3CO_2H \cdot 1/2H_2O$<br>Calcd.% :C, 54.54; H, 4.76; N, 12.72<br>Found% :C, 54.27; H, 4.79; N, 12.72 |
| 95 | 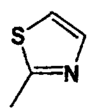 | pale yellow needles[trifluoroacetate](EtOH)<br>mp, 243-245°C(decomposition)<br>Elemental analysis for $C_{22}H_{22}F_3N_5S \cdot CF_3CO_2H$<br>Calcd.% :C, 51.52; H, 4.14; N, 12.52<br>Found% :C, 51.61; H, 3.98; N, 12.70 |
| 96 | 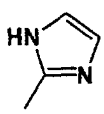 | colorless needles[trifluoroacetate](EtOH)<br>mp, 306-308°C(decomposition)<br>Elemental analysis for<br>$C_{22}H_{23}F_3N_6 \cdot CF_3CO_2H \cdot 1/4H_2O$<br>Calcd.% :C, 52.70; H, 4.51; N, 15.36<br>Found% :C, 52.79; H, 4.33; N, 15.37 |
| 97 | 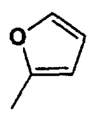 | pale brown needles[trifluoroacetate](EtOH)<br>mp, 232-234°C(decomposition)<br>Elemental analysis for<br>$C_{23}H_{23}F_3N_4O \cdot CF_3CO_2H \cdot 1/2H_2O$<br>Calcd.% :C, 54.45; H, 4.57; N, 10.16<br>Found% :C, 54.57; H, 4.37; N, 10.24 |
| 98 | 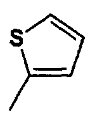 | pale yellow needles[trifluoroacetate](EtOH)<br>mp, 227-229°C(decomposition)<br>Elemental analysis for<br>$C_{23}H_{23}F_3N_4S \cdot CF_3CO_2H \cdot 1/4H_2O$<br>Calcd.% :C, 53.33; H, 4.39; N, 9.95<br>Found% :C, 53.32; H, 4.27; N, 9.76 |

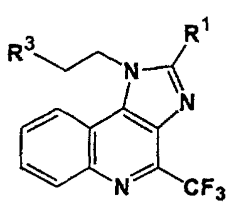

| Example | $-R^1$ | $R^3-$ | Physical properties [salt] (Recrystallization solvent) |
|---|---|---|---|
| 99 | -Ph | 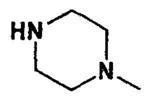 | pale brown crystals(AcOEt)<br>mp,187-188℃<br>Elemental analysis for $C_{23}H_{22}F_3N_5$<br>Calcd.% :C, 64.93; H, 5.21; N, 16.46<br>Found% :C, 64.84; H, 5.26; N, 16.38 |
| 100 | 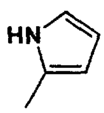 | 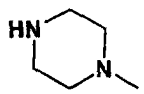 | colorless needles[trifluoroacetate] (EtOH)<br>mp,209-210.5℃<br>Elemental analysis for $C_{21}H_{21}F_3N_6 \cdot CF_3CO_2H$<br>Calcd.% :C, 52.27; H, 4.20; N, 15.90<br>Found% :C, 52.00; H, 4.17; N, 15.98 |
| 101 | -Ph | 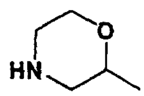 | colorless needles[trifluoroacetate] (EtOH)<br>mp,224-228℃(decomposition)<br>Elemental analysis for<br>$C_{23}H_{21}F_3N_4O \cdot CF_3CO_2H \cdot 1/4H_2O$<br>Calcd.% :C, 55.10; H, 4.16; N, 10.28<br>Found% :C, 54.84; H, 4.13; N, 10.34 |
| 102 | 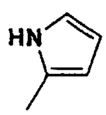 | 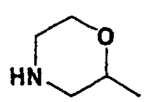 | pale yellow plates[trifluoroacetate] (EtOH)<br>mp,254-258℃(decomposition)<br>Elemental analysis for $C_{21}H_{20}F_3N_5O \cdot CF_3CO_2H$<br>Calcd.% :C, 52.18; H, 4.00; N, 13.23<br>Found% :C, 52.10; H, 4.16; N, 13.45 |
| 103 | -Ph | 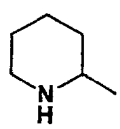 | colorless crystals[trifluoroacetate]<br>(AcOEt-EtOH)<br>mp, 229.5-231.5℃(decomposition)<br>Elemental analysis for $C_{24}H_{23}F_3N_4 \cdot CF_3CO_2H$<br>Calcd.% :C, 57.99; H, 4.49; N, 10.40<br>Found% :C, 57.94; H, 4.42; N, 10.46 |
| 104 | 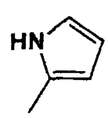 | 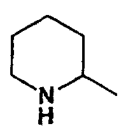 | colorless crystals[trifluoroacetate](MeOH)<br>mp, 238.5-240℃(decomposition)<br>Elemental analysis for $C_{22}H_{22}F_3N_5 \cdot CF_3CO_2H$<br>Calcd.% :C, 54.65; H, 4.40; N, 13.28<br>Found% :C, 54.61; H, 4.25; N, 13.41 |

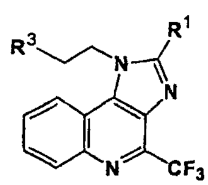

| Example | $-R^1$ | $R^3-$ | Physical properties [salt] (Recrystallization solvent) |
|---|---|---|---|
| 105 | 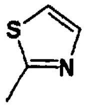 | 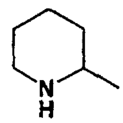 | colorless crystals[trifluoroacetate] (MeOH)<br>mp, 235.5-237.5°C (decomposition)<br>Elemental analysis for<br>$C_{21}H_{20}F_3N_5S \cdot CF_3CO_2H$<br>Calcd.% :C, 50.64; H, 3.88; N, 12.84<br>Found% :C, 50.53; H, 3.79; N, 12.90 |
| 106 | -Ph | 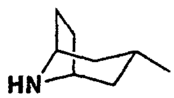 | colorless needles[trifluoroacetate] (EtOH)<br>mp, 249-251°C (decomposition)<br>Elemental analysis for<br>$C_{26}H_{25}F_3N_4 \cdot CF_3CO_2H$<br>Calcd.% :C, 59.57; H, 4.64; N, 9.92<br>Found% :C, 59.27; H, 4.91; N, 9.99 |
| 107 | 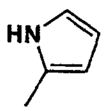 | 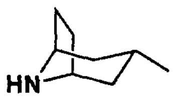 | pale yellow needles[trifluoroacetate] (EtOH)<br>mp, 263-265°C (decomposition)<br>Elemental analysis for<br>$C_{24}H_{24}F_3N_5 \cdot CF_3CO_2H$<br>Calcd.% :C, 56.42; H, 4.55; N, 12.65<br>Found% :C, 56.22; H, 4.59; N, 12.62 |
| 108 | -Ph | $H_2N-$ | colorless crystals(AcOEt)<br>mp, 174.5-175.5°C<br>Elemental analysis for $C_{19}H_{15}F_3N_4$<br>Calcd.% :C, 64.04; H, 4.24; N, 15.72<br>Found% :C, 63.89; H, 4.25; N, 15.67 |
| 109 | 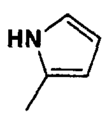 | $H_2N-$ | pale brown crystals(AcOEt)<br>mp, 195.5-196.5°C<br>Elemental analysis for $C_{17}H_{14}F_3N_5$<br>Calcd.% :C, 59.13; H, 4.09; N, 20.28<br>Found% :C, 59.09; H, 4.14; N, 20.19 |

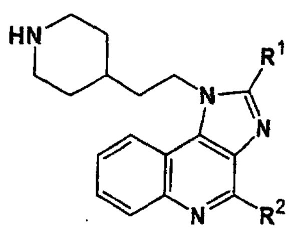

| Example | $-R^1$ | $-R^2$ | Physical properties [salt] (Recrystallization solvent) |
|---|---|---|---|
| 110 | -Ph | 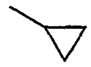 | pale brown crystals[fumarate] (MeOH)<br>mp, 142.5-147°C(decomposition)<br>Elemental analysis for<br>$C_{26}H_{28}N_4 \cdot 1/2C_4H_4O_4 \cdot 3/4H_2O$<br>Calcd.% :C, 71.85; H, 6.78; N, 11.97<br>Found% :C, 71.92; H, 6.61; N, 11.91 |
| 111 | 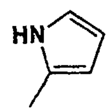 | 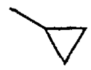 | pale brown needles(EtOH)<br>mp, 212-213.5°C<br>Elemental analysis for $C_{24}H_{27}N_5 \cdot 1/2H_2O$<br>Calcd.% :C, 73.07; H, 7.15; N, 17.75<br>Found% :C, 73.17; H, 7.04; N, 17.59 |
| 112 | -Ph | -Ph | colorless needles(MeOH-EtOH)<br>mp, 209-211°C<br>Elemental analysis for $C_{29}H_{28}N_4$<br>Calcd.% :C, 80.52; H, 6.52; N, 12.95<br>Found% :C, 80.26; H, 6.57; N, 12.92 |
| 113 | 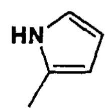 | -Ph | pale brown crystals(DMF-$H_2O$)<br>mp, 268-270°C<br>Elemental analysis for $C_{27}H_{27}N_5 \cdot 1/2H_2O$<br>Calcd.% :C, 75.32; H, 6.56; N, 16.27<br>Found% :C, 75.12; H, 6.43; N, 16.36 |
| 114 | -Ph | 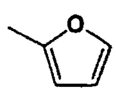 | colorless crystals(MeOH)<br>mp, 224.5-225.5°C<br>Elemental analysis for $C_{27}H_{26}N_4O$<br>Calcd.% :C, 76.75; H, 6.20; N, 13.26<br>Found% :C, 76.76; H, 6.19; N, 13.22 |
| 115 | -Ph | 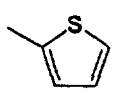 | colorless crystals(MeOH-$ClCH_2CH_2Cl$)<br>mp, 210-212°C<br>Elemental analysis for $C_{27}H_{26}N_4S$<br>Calcd.% :C, 73.94; H, 5.98; N, 12.77<br>Found% :C, 73.91; H, 5.92; N, 12.67 |

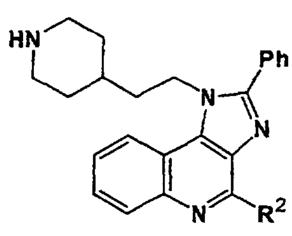

| Example | $R^2$ | Physical properties [salt] (Recrystallization solvent) |
|---|---|---|
| 116 | SH | colorless crystals[trifluoroacetate](MeOH-DMF) mp, 251-255°C(decomposition)<br>Elemental analysis for $C_{23}H_{24}N_4S \cdot CF_3CO_2H \cdot 1/4H_2O$<br>Calcd.% :C, 59.22; H, 5.07; N, 11.05<br>Found% :C, 59.07; H, 5.05; N, 11.29 |

(continued)

| Example | $R^2$ | Physical properties [salt] (Recrystallization solvent) |
|---|---|---|
| 117 | CN | colorless crystals[trifluoroacetate](MeOH-2-PrOH) mp, 245-251°C (decomposition)<br>Elemental analysis for $C_{24}H_{23}N_5 \cdot CF_3CO_2H \cdot 1/4H_2O$<br>Calcd.% :C, 62.46; H, 4.94; N, 14.01<br>Found% :C, 62.63; H, 4.88; N, 14.22 |
| 118 | $CO_2H$ | colorless crystals[trifluoroacetate](MeOH) mp, 212-215°C<br>Elemental analysis for $C_{24}H_{24}N_4O_2 \cdot CF_3CO_2H \cdot 1/4H_2O$<br>Calcd.% :C, 60.17; H, 4.95; N, 10.80<br>Found% :C, 59.98; H, 5.14; N, 11.03 |

Example 119

N-[2-(2-Phenyl-4-trifluoromethyl-1H-imidazo[4,5-c]quinolin-1-yl)ethyl]acetamide

To a solution of 0.50 g of 1-(2-aminoethyl)-2-phenyl-4-trifluoromethyl-1H-imidazo[4,5-c]quinoline in 3 ml of pyridine, 0.28 ml of acetic anhydride was added dropwise, and the mixture was stirred at room temperature for 6 hours. The reaction mixture was added with water, and adjusted to pH 4 with 10% hydrochloric acid. The precipitated crystals were collected with filtration, and washed with successively with water and diisopropyl ether to give 0.45 g of colorless crystals. Recrystallization from ethyl acetate gave colorless crystals having the melting point of from 217 to 218°C.
Elemental analysis for $C_{21}H_{17}F_3N_4O$
Calculated % C,63.31; H,4.30; N,14.06
Found % C,63.46; H,4.32; N,14.10

Example 120

N-[2-(2-Phenyl-4-trifluoromethyl-1H-imidazo[4,5-c]quinolin-1-yl)ethyl]methanesulfonamide

To a solution of 0.50 g of 1-(2-aminoethyl)-2-phenyl-4-trifluoromethyl-1H-imidazo[4,5-c]quinoline and 0.2 ml of triethylamine in 5 ml of tetrahydrofuran, 0.11 ml of methanesulfonyl chloride was added, and the mixture was stirred at room temperature for 2 hours. The reaction mixture was added with water, and the precipitated crystals were collected by filtration, and washed successively with water and diisopropyl ether to give 0.49 g of colorless crystals. Recrystallization from ethyl acetate gave colorless crystals having the melting point of from 224.5 to 225°C.
Elemental analysis for $C_{20}H_{17}F_3N_4O_2S$
Calculated % C,55.29; H,3.94; N,12.90
Found % C,55.30; H,3.94; N,12.70

In accordance with the method of Example 120, the compounds of Examples 121 through 125 were obtained.

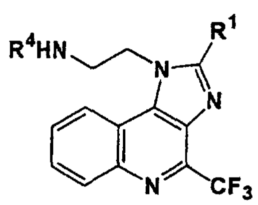

| Example | $-R^1$ | $R^4$ | Physical properties (Recrystallization solvent) |
|---|---|---|---|
| 121 | -Ph | PhCO | colorless crystals(AcOEt)<br>mp, 256-256.5°C<br>Elemental analysis for $C_{26}H_{19}F_3N_4O$<br>Calcd.% :C, 67.82; H, 4.16; N, 12.17<br>Found% :C, 67.86; H, 4.19; N, 12.18 |
| 122 | -Ph | $EtO_2C$ | colorless crystals(AcOEt)<br>mp, 232.5-233.5°C<br>Elemental analysis for $C_{22}H_{19}F_3N_4O_2$<br>Calcd.% :C, 61.68; H, 4.47; N, 13.08<br>Found% :C, 61.74; H, 4.53; N, 13.04 |
| 123 | -Ph | 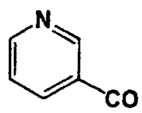 | colorless crystals(AcOEt)<br>mp, 227.5-228.5°C<br>Elemental analysis for $C_{25}H_{18}F_3N_5O$<br>Calcd.% :C, 65.07; H, 3.93; N, 15.18<br>Found% :C, 65.05; H, 4.01; N, 15.13 |
| 124 | -Ph | $ClCH_2CO$ | colorless crystals(AcOEt)<br>mp, 230.5-231.5°C<br>Elemental analysis for $C_{21}H_{16}ClF_3N_4O$<br>Calcd.% :C, 58.27; H, 3.73; N, 12.94<br>Found% :C, 58.23; H, 3.78; N, 12.89 |
| 125 | 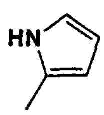 | $ClCH_2CO$ | pale brown crystals(AcOEt)<br>mp, 234-235°C<br>Elemental analysis for $C_{19}H_{15}ClF_3N_5O$<br>Calcd.% :C, 54.10; H, 3.58; N, 16.60<br>Found% :C, 54.15; H, 3.61; N, 16.63 |

Example 126

2-Methylamino-N-[2-[2-(2-pyrrolyl)-4-trifluoromethyl-lH-imidazo[4,5-c]quinolin-1-yl]ethyl]acetamide

To 0.30 g of 2-chloro-N-[2-[2-(2-pyrrolyl)-4-trifluoromethyl-1H-imidazo[4,5-c]quinolin-1-yl]ethyl]acetamide, 6 ml of 40 % methanol solution of methylamine was added, and the mixture was stirred at room temperature for 4 hours. After the reaction, the solvent was evaporated, and the residue was added with saturated aqueous solution of sodium hydrogencarbonate, and extracted with ethyl acetate. The extract was washed with saturated brine, and dried, and the solvent was evaporated, and the obtained residue was washed with diisopropyl ether to give 0.25 g of pale brown crystals. Recrystallization from ethyl acetate gave colorless crystals having the melting point of from 217.5 to 219.5°C.
Elemental analysis for $C_{20}H_{19}F_3N_6O$
Calculated % C,57.69; H,4.60; N,20.18
Found % C,57.67; H,4.50; N,20.15

In accordance with the method of Example 126, the compounds of Examples 127 through 129 were obtained.

Example 127

2-Methylamino-N-[2-(2-phenyl-4-trifluoromethyl-1H-imidazo[4,5-c]quinolin-1-yl)ethyl]acetamide

Appearance : colorless crystals
Recrystallization solvent: ethyl acetate
Melting point : 208-209°C
Elemental analysis for $C_{22}H_{20}F_3N_5O$
Calculated % C,61.82; H,4.72; N,16.39
Found % C,61.60; H,4.76; N,16.17

Example 128

2-Dimethylamino-N-[2-(2-phenyl-4-trifluoromethyl-1H-imidazo[4,5-c]quinolin-1-yl)ethyl]acetamide fumarate

Appearance : colorless crystals
Recrystallization solvent: methanol
Melting point : 207.5-208°C
Elemental analysis for $C_{23}H_{22}F_3N_5O \cdot 3/2C_4H_4O_4$
Calculated % C,56.58; H,4.58; N,11.38
Found % C,56.52; H,4.70; N,11.57

Example 129

2-Dimethylamino-N-[2-[2-(2-pyrrolyl)-4-trifluoromethyl-1H-imidazo[4,5-c]quinolin-1-yl]ethyl]acetamide fumarate

Appearance : pale brown crystals
Recrystallization solvent : methanol
Melting point : 219-222°C (decomposition)
Elemental analysis for $C_{21}H_{21}F_3N_6O \cdot 1/2C_4H_4O_4$
Calculated % C,56.55; H,4.75; N,17.21
Found % C,56.58; H,4.80; N,17.18

Example 130

2-Amino-N-[2-(2-phenyl-4-trifluoromethyl-1H-imidazo[4,5-c]quinolin-1-yl)ethyl]acetamide

(1)N-[2-(2-Phenyl-4-trifluoromethyl-lH-imidazo[4,5-c]quinolin-1-yl)ethyl]-2-phthalimidoacetamide

A solution of 0.40 g of 2-chloro-N-[2-(2-phenyl-4-trifluoromethyl-1H-imidazo[4,5-c]quinolin-1-yl)ethyl]acetamide and 0.17 g of potassium phthalimide in 4 ml of N,N-dimethylformamide was stirred at 60°C for 16 hours. The reaction mixture was added with water, and the precipitated crystals were collected by filtration, and washed successively with water and diisopropyl ether. The crystals were purified by silica gel column chromatography using a mixture of ethyl acetate and n-heptane (2:1) as eluting solvent, and washed with diisopropyl ether to give 0.37 g of colorless crystals. Recrystallization from ethyl acetate gave colorless crystals having the melting point of from 233 to 233.5°C.
Elemental analysis for $C_{29}H_{20}F_3N_5O_3$
Calculated % C,64.09; H,3.71; N,12.89
Found % C,64.04; H,3.93; N,12.92

(2)2-Amino-N-[2-(2-phenyl-4-trifluoromethyl-1H-imidazo[4,5-c]quinolin-1-yl)ethyl]acetamide

A solution of 0.30 g of N-[2-(2-phenyl-4-trifluoromethyl-1H-imidazo[4,5-c]quinolin-1-yl)ethyl]-2-phthalimidoacetamide and 0.03 ml of 90 % hydrazine hydrate in 3 ml of ethanol was refluxed for 6 hours. After the reaction, the solvent was evaporated, and the residue was added with water, and adjusted to pH 9 with potassium carbonate, and extracted with ethyl acetate. The extract was washed with saturated brine, and dried, and the solvent was evaporated. The residue was washed with diisopropyl ether to give 0.19 g of colorless crystals. Recrystallization from ethyl acetate gave colorless crystals having the melting point of from 188 to 189°C.
Elemental analysis for $C_{21}H_{18}F_3N_5O$
Calculated % C,61.01; H,4.39; N,16.94
Found % C,60.96; H,4.36; N,16.94
In accordance with the method of Example 130, the compound of Example 131 was obtained.

Example 131

2-Amino-N-[2-[2-(2-pyrrolyl)-4-trifluoromethyl-1H-imidazo[4,5-c]quinolin-1-yl]ethyl]acetamide fumarate

(1)2-Phthalimido-N-[2-[2-(2-pyrrolyl)-4-trifluoromethyl-1H-imidazo[4,5-c]quinolin-1-yl]ethyl]acetamide

Appearance : pale brown crystals
Recrystallization solvent: ethyl acetate
Melting point : 278.5-280.5°C(decomposition)
Elemental analysis for $C_{27}H_{19}F_3N_6O_3$
Calculated % C,60.90; H,3.60; N,15.78
Found % C,60.76; H,3.74; N,15.71

(2)2-Amino-N-[2-[2-(2-pyrrolyl)-4-trifluoromethyl-1H-imidazo[4,5-c]quinolin-1-yl]ethyl]acetamide fumarate

Appearance : pale brown crystals
Recrystallization solvent : methanol
Melting point : 176-177°C(decomposition)
Elemental analysis for $C_{19}H_{17}F_3N_6O \cdot C_4H_4O_4 \cdot 1/4H_2O0$
Calculated % C,52.82; H,4.14; N,16.07
Found % C,52.71; H,4.21; N,16.31

As an example of the excellent effects of the compounds of the present invention, experimental results of inhibitory actions against production of TNF-alpha and IL-1 beta in human cells will be shown below.

1. Preparation of blood cells for culture

About 50 mL of whole blood was collected from each adult healthy volunteer by venepuncture, and poured into plastic tubes which containing 170 micro L of Novo-heparin 1000 (produced by Novo-Nordisk). Then, PBMCs (Peripheral Blood Mononuclear Cells) were separated using LeucoPREP™ (produced by Becton Dickinson) lymphocyte separation tube, and cultured with RPMI-1640 medium (produced by Nissui Pharmaceutical Co.) containing 2 mM L-glutamine (produced by Life Technologies), 2.5 U/mL penicillin-2.5 micro g/mL streptomycin solution (produced by Life Technologies) supplemented with 10 % fetal calf serum (produced by Intergen Company) at 1 x $10^6$ cells/mL.

2. Preparation of test compounds

Test compounds were dissolved in distilled ultra-pure water, dimethyl sulfoxide or 0.1 M hydrochloric acid at 20 micro M as far as possible, and then sequentially diluted with saline and used.

3.Treatment of cells with medicaments

A hundred and eighty micro L of prepared human PBMCs were added to each well of 96-well plate (MicroTest III™ tissue culture plate; produced by Becton Dickinson) containing 10 micro L of adequate concentrations solution of test compounds. Then 30 minutes after, 10 micro L of 20 micro g/mL lipopolysaccharide (LPS) was added to each well, and cells in the well plate were covered with plastic lid and incubated for 16 hours at 37°C in an atmosphere of 5 % $CO_2$.

4. Measurement of human TNF-alpha and human IL-1 beta

The levels of human TNF-alpha and human IL-1 beta in culture supernatant were measured by constructed sandwich enzyme-linked immunosorbent assay method. Ninety-six-well microtiter plates were coated with diluted anti-cytokine antibody (capture/first-antibody). After washing the wells, the culture supernatant was diluted appropriately, added to each well and incubated. Then detection/second-antibody against cytokine and HRP (horseradish peroxidase)-conjugated/third-antibody against detection-antibody were added to wells sequentially, with interposed washing processes. After the final washing process, reaction of colorimetric quantification was started by addition of tetramethylbenzidine solution (produced by DAKO) to each well. After quenching the reaction by addition of 0.5 M sulfuric acid, optical absorbance of each well at 450 nm was measured by M-VmaxTM kinetic microplate reader (produced by Molecular Devices). Concentrations of cytokines were determined by Softmax™ (produced by Molecular Devices) quantification software in comparison with calibration curve derived from corresponding recombinant cytokines as standard. For measurement of human TNF-alpha, monoclonal mouse anti-human TNF-alpha (produced by ENDOGEN), polyclonal rabbit anti-human TNF-alpha (produced by Pharma Biotechnologie Hannover), peroxidase-conjugated donkey anti-rabbit IgG antibodies (produced by Jackson ImmunoRes. Labs.), and recombinant human TNF-alpha (produced by INTERGEN Company) were used for the capture-, detection-, HRP-conjugated-antibodies and the standard for calibration curve, respectively. In the case of measuring human IL-1 beta, monoclonal anti-human IL-1 beta (produced by Cistron), polyclonal sheep anti-human IL-1 beta (produced by Biogenesis), HRP-conjugated donkey anti-goat IgG antibodies (produced by Chemicon International) and recombinant human IL-1 beta (produced by R&D Systems) were used for the capture-, detection-, HRP-conjugated-antibodies and standard for calibration curve, respectively.

The activities of test compounds on both TNF-alpha and IL-1 beta are shown as inhibitory percentages of cytokine production based on the following equation.

$$\text{Inhibitory percentage(\%)} = \left(1 - \frac{\text{Cytokine production in test compounds-treated cells}}{\text{Cytokine production in solvent-treated cells}}\right) \times 100$$

Results are shown in Table 32 and Table 33.

Table 32 :

| Inhibitory action against TNF-alpha production in human cells | | | | | |
|---|---|---|---|---|---|
| Test compounds | Administered concentration (micro mol/L) | | | | |
| | 0.01 | 0.03 | 0.10 | 0.3 | 1.0 |
| Example 102 | 96 | 84 | 25 | 23 | 27 |

Table 33 :

| Inhibitory action against IL-1 beta production in human cells | | | | | |
|---|---|---|---|---|---|
| Test compounds | Administered concentration (micro mol/L) | | | | |
| | 0.01 | 0.03 | 0.10 | 0.3 | 1.0 |
| Example 102 | 81 | 67 | 48 | 0 | 0 |

These results clearly indicate that the compounds of the present invention have excellent inhibitory actions against production of TNF-alpha and IL-1 beta.

Industrial Applicability

The compounds of the present invention have excellent inhibitory action against production of TNF or IL-1, and they are useful as medicaments for preventive or therapeutic treatment of diseases in which a cytokine is involved.

## Claims

**1.** A 1H-imidazopyridine derivative represented by the following general formula (I) or a salt thereof:

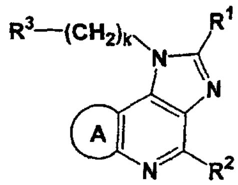

(I)

wherein $R^1$ represents hydrogen atom, an alkyl group which may be substituted, a cycloalkyl group which may be substituted, or an aryl group which may be substituted; $R^2$ represents a cycloalkyl group which may be substituted, an alkyl group which may be substituted, an aryl group which may be substituted, cyano group, mercapto group, carboxyl group, or carbamoyl group; ring A represents a homocyclic or heterocyclic ring which may be substituted; $R^3$ represents an amino group which may be substituted or a saturated nitrogen-containing heterocyclic group which may be substituted; and k represents an integer of from 0 to 3; provided that the compound wherein $R^3$ represents a saturated nitrogen-containing heterocyclic group which may be substituted and $R^2$ is a non-substituted alkyl group is excluded.

**2.** A 1H-imidazopyridine derivative represented by the following general formula (II) or a salt thereof:

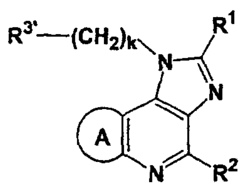

(II)

wherein $R^1$ represents hydrogen atom, an alkyl group which may be substituted, a cycloalkyl group which may be substituted, or an aryl group which may be substituted; $R^2$ represents a cycloalkyl group which may be substituted, an alkyl group which may be substituted, an aryl group which may be substituted, cyano group, mercapto group, carboxyl group, or carbamoyl group; ring A represents a homocyclic or heterocyclic ring which may be substituted; k represents an integer of from 0 to 3; $R^{3'}$ represents a group represented by the following general formula (III)

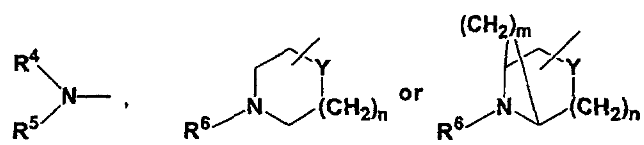

(III)

$R^4$, $R^5$, $R^6$ may be the same or different and represent hydrogen atom, an alkyl group which may be substituted, a benzyl group which may be substituted, triphenylmethyl group, an acyl group which may be substituted, an alkoxycarbonyl group which may be substituted, a benzyloxycarbonyl group which may be substituted, a thiocarbamoyl group which may be substituted, an alkanesulfonyl group which may be substituted, a benzenesulfonyl group which may be substituted, or an amidino group which may be substituted; Y represents oxygen atom, sulfur atom, or nitrogen atom, a group represented by $CH_2$, CH, or NH, or a single bond; and m and n may be the same or different and represent an integer of from 0 to 2, provided that when $R^{3'}$ represents a group represented by the following general formula (IV):

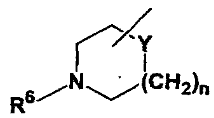 or 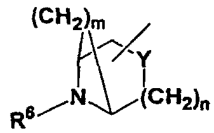 (IV)

$R^2$ does not represent a non-substituted alkyl group.

**3.** The compound or a salt thereof according to claim 1 or claim 2, wherein the ring A is a benzene ring which may be substituted or a thiophene ring which may be substituted.

**4.** The compound or a salt thereof according to any one of claims 1 to 3, wherein $R^2$ is trifluoromethyl group.

**5.** A medicament which comprises the compound or a pharmacologically acceptable salt thereof as an active ingredient according to any one of claims 1 to 4.

**6.** The medicament according to claim 5, which is used for preventive and/or therapeutic treatment of a disease in which a cytokine is involved.

**7.** The medicament according to claim 6, wherein the cytokine is tumor necrotizing factor (TNF) or interleukin-1 (IL-1).

**8.** An inhibitor against production of a cytokine which comprises the compound or a pharmacologically acceptable salt thereof as an active ingredient according to any one of claims 1 to 4.

**9.** A method for a preventive and/or therapeutic treatment of a disease in which a cytokine is involved, which comprises the step of administering a preventively and/or therapeutically effective amount of the compound or a pharmacologically acceptable salt thereof according to any one of claims 1 to 4 to a mammal including a human.

**10.** A use of the compound or a pharmacologically acceptable salt thereof according to any one of claims 1 to 4 for manufacture of the medicament according to any one of claims 5 to 7.

## INTERNATIONAL SEARCH REPORT

International application No.
PCT/JP01/00816

A. CLASSIFICATION OF SUBJECT MATTER
Int.Cl$^7$ C07D471/04, A61K31/437, A61P3/10, 11/06, 17/00, 25/00, 29/00, 31/04, 37/06, 37/08, 43/00

According to International Patent Classification (IPC) or to both national classification and IPC

B. FIELDS SEARCHED

Minimum documentation searched (classification system followed by classification symbols)
Int.Cl$^7$ C07D471/04, A61K31/437

Documentation searched other than minimum documentation to the extent that such documents are included in the fields searched

Electronic data base consulted during the international search (name of data base and, where practicable, search terms used)
CAPLUS(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | WO, 00/09506, A1 (HOKURIKU SEIYAKU CO., LTD.), 24 February, 2000 (24.02.00) & AU, 5197499, A & JP, 2000-119271, A | 1-8,10 |
| A | WO, 92/15581, A1 (MINNESOTA MINING AND MANUFACTURING COMPANY), 17 September, 1992 (17.09.92) & US, 5175296, A & CA, 2104781, A & KR, 226184, B & JP, 11-269177, A & AU, 657958, B & HU, 66968, A | 1-8,10 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 April, 2001 (24.04.01) | 01 May, 2001 (01.05.01) |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP01/00816

**Box I Observations where certain claims were found unsearchable (Continuation of item 1 of first sheet)**

This international search report has not been established in respect of certain claims under Article 17(2)(a) for the following reasons:

1. ☒ Claims Nos.: 9
because they relate to subject matter not required to be searched by this Authority, namely:

The invention as set forth in claim 9 pertains to methods for treatment of the human body by therapy.

2. ☒ Claims Nos.: 1-8,10
because they relate to parts of the international application that do not comply with the prescribed requirements to such an extent that no meaningful international search can be carried out, specifically:

(See extra sheet.)

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)

**INTERNATIONAL SEARCH REPORT**

International application No.
PCT/JP01/00816

Continuation of Box No.I-2 of continuation of first sheet(1)

The technical features of the inventions as set forth in claims 1 to 8 and 10 reside in the compounds represented by the general formula (I) per se or use of these compounds as drugs.

Concerning the ring A in the general formula (I), it is stated in claim 1 "represents an optionally substituted homocycle or heterocycle".

Although the expression "represents an optionally substituted homocycle or heterocycle" seemingly involves cyclic factors over an extremely broad scope, no compounds but those wherein the ring A is a benzene ring are disclosed in practice, in a state substantially supported by production examples and the like, in the description.

Therefore, the above-described claims and the description fail to satisfy the definite requirements to such an extent as enabling any meaningful international search.

In this report, compounds wherein the ring A is an optionally substituted benzene ring have been examined by taking the contents of the description into consideration.

Form PCT/ISA/210 (extra sheet) (July 1992)